# EUROPEAN PATENT APPLICATION

(11) **EP 4 332 237 A1**
(43) Date of publication of application: **06.03.2024**
(21) Application number: 21940721.0
(22) Date of filing: 18.05.2021
(51) Int. Cl.: C12Q 1/6865, C12N 15/09

(54) **ISOTHERMAL GENE AMPLIFICATION METHOD, GENE DETECTION METHOD, VIRUS DETECTION METHOD, AND KIT USED THEREFOR**

(71) Applicant: Bioseeds Corporation, Nomi-shi, Ishikawa, 923-1211 (JP)
(72) Inventor: BIYANI Manish, Nomi-shi, Ishikawa 923-1211 (JP)
(74) Representative: Hoffmann Eitle
(86) International application number: PCT/JP2021/018799
(87) International publication number: WO 2022/244104

(57) **Abstract**

An isothermal gene amplification method that allows an RNA virus to be detected easily and inexpensively in a short time is presented. The isothermal gene amplification method involves a target sequence in an RNA genome, and includes: (A) producing an amplification reaction template; (B) DNA amplifying for increasing the amplification reaction template; and (C) RNA amplifying.

## Description

### TECHNICAL FIELD

The present invention relates to an isothermal gene amplification method, a gene detection method, a virus detection method, and kits for use therein.

### BACKGROUND ART

In recent years, RNA viral infections such as human and avian influenza, severe acute respiratory syndrome (SARS), and the like have occurred, and in the last few years, worldwide infection (pandemic) of a new type of coronavirus (SARS-CoV-2) has become a global issue. Detecting RNA viruses is essential to prevent the spread of such RNA viral infections. Patent Literature 1 discloses a primer set for determining an NA subtype of avian influenza virus by a PCR method.

### Citation List

### Patent Literature

Patent Literature 1: JP 2010-252659 A

### SUMMARY OF INVENTION

### Technical Problem

However, the PCR method has the problem of being time-consuming, labor-intensive, and expensive because it requires a special device such as a thermal cycler for raising or lowering the temperature of a reaction solution.

Accordingly, it is an object of the present invention to provide a novel isothermal gene amplification method that allows an RNA of a pathogen, such as a virus of an infectious disease, to be detected easily and inexpensively in a short time.

### Solution to Problem

In order to achieve the above object, the present invention provides an isothermal gene amplification method of a target sequence in an RNA genome (hereinafter, also referred to as an "amplification method"), including:
(A) producing an amplification reaction template, including:
   (A1) hybridizing, a first primer having a promoter sequence of an RNA polymerase on its 5' side and a complementary sequence that is complementary to a 5'-side sequence of the target sequence in the RNA genome on its 3' side, to the 5'-side sequence of the target sequence in the RNA genome, and extending a 3' end of the first primer by reverse transcription activity of a reverse transcriptase to produce a double-stranded complex of the RNA genome and an extended DNA strand;
   (A2) degrading the RNA genome of the double-stranded complex formed in (A1) by RNA degrading activity of the reverse transcriptase to produce a single-stranded DNA having a first primer sequence; and
   (A3) hybridizing a second primer having the same sequence as a 3'-side sequence of the target sequence to a sequence complementary to the target sequence of the single-stranded DNA produced in (A2), and extending a 3' end of the second primer by DNA synthetic activity of the reverse transcriptase to produce a single-stranded DNA having a second primer sequence, thereby producing a double-stranded DNA as the amplification reaction template;
(B) DNA amplifying for increasing the amplification reaction template, including:
   (B 1) hybridizing the second primer to a 3' side of the single-stranded DNA including the first primer and hybridizing the first primer to a 3' side of the single-stranded DNA having the second primer sequence in the double-stranded DNA as the amplification reaction template produced in (A3) by a recombinase in a presence of a single-stranded DNA-binding protein; and
   (B2) extending the 3' end of the first primer and the 3' end of the second primer by strand displacement DNA synthetic activity of a strand displacement DNA polymerase in a presence of the single-stranded DNA-binding protein to produce a double-stranded DNA that is the same as the amplification reaction template produced in (A3); and
(C) RNA amplifying, including:
   (C1) synthesizing a single-stranded RNA by RNA synthetic activity of an RNA polymerase with a sequence that is downstream on a 3'-side relative to the promoter sequence of the double-stranded DNA as the amplification reaction template produced in (A3) being used as a template sequence;
   (C2) hybridizing the second primer to a 5' side of the single-stranded RNA synthesized in (C1), and extending the 3' end of the second primer by the reverse transcription activity of the reverse transcriptase to produce a double-stranded complex of the single-stranded RNA and an extended DNA;
   (C3) degrading the single-stranded RNA of the double-stranded complex produced in (C2) by the RNA degrading activity of the reverse transcriptase to produce a single-stranded DNA; and
   (C4) hybridizing the first primer to a 3' side of the single-stranded DNA produced in (C3), extending the 3' end of the first primer by the DNA synthetic activity of the reverse transcriptase, and extending a 3' end of the single-stranded DNA produced in (C3) with the promoter sequence of the RNA polymerase of the first primer being used as a template to produce a double-stranded DNA the same as the amplification reaction template produced in (A3).

The present invention also provides a kit for use in the isothermal gene amplification method according to the present invention (hereinafter, also referred to as a "first kit"), including:
(a1) the reverse transcriptase;
(a2) the first primer;
(a3) the second primer;
(b 1) the recombinase;
(b2) the single-stranded DNA-binding protein;
(b3) the strand displacement DNA polymerase; and
(c1) the RNA polymerase.

The present invention also provides a gene detection method for detecting a gene of a target sequence of an RNA genome, including:
amplifying a gene; and
detecting an amplified gene, wherein
the amplifying is performed by the isothermal gene amplification method according to the present invention.

The present invention also provides a kit for use in the gene detection method according to the present invention (hereinafter, also referred to as a "second kit"), including:
(a1) the reverse transcriptase;
(a2) the first primer;
(a3) the second primer;
(b 1) the recombinase;
(b2) the single-stranded DNA-binding protein;
(b3) the strand displacement DNA polymerase;
(c1) the RNApolymerase;
(d1) the immobilized antibody;
(d2) a substrate; and
(d3) a labeling substance.

The present invention also provides an RNA virus detection method being a method for detecting a sequence specific to the RNA virus in a viral genome as a target sequence, wherein
the detection of the target sequence is performed by the gene detection method according to the present invention.

The present invention also provides a kit for use in the RNA virus detection method according to the present invention (hereinafter, also referred to as a "third kit"), including:
the first kit or the second kit.

### Advantageous Effects of Invention

According to the present invention, it is possible to easily and inexpensively detect a pathogen such as a virus of an infectious disease in a short time.

### BRIEF DESCRIPTION OF DRAWINGS

[FIG. 1] FIG. 1 is a schematic diagram showing the mechanism and main steps of the isothermal gene amplification method of the present invention.
[FIG. 2] FIG. 2 is a schematic diagram showing an example of main steps in the first gene detection method of the present invention.
[FIG. 3] FIG. 3 is a diagram showing an example of the electrochemical detection method in the second gene detection method of the present invention.
[FIG. 4] FIG. 4 is a schematic diagram showing the target sequence and primers in Example 1.
[FIG. 5] FIG. 5 is a photograph showing a gel image of the amplified products after the reaction in Example 1.
[FIG. 6] FIG. 6 is a photograph showing a gel image of the amplified products after the reaction in Example 1.
[FIG. 7] FIG. 7 is a photograph showing a gel image of the amplified products after the reaction in Example 1.
[FIG.8] FIG.8 is a schematic diagram showing the target sequence and primers in Example 2.
[FIG. 9] FIG. 9 is a photograph showing a gel image of the amplified products after the reaction in Example 2.
[FIGs. 10A and 10B] FIG. 10A is a schematic diagram for explaining the configuration of the LFD in Example 3, and FIG. 10B is a photograph showing the results of the lateral flow immunoassay in Example 3.
[FIG. 11] FIG. 11 is a schematic diagram showing the target sequence and primers in Example 4.
[FIGs. 12A and 12B] FIG. 12A is a schematic diagram showing steps of the reaction in Example 4, and FIG. 12B is a photograph showing a gel image of the amplified products after the reaction in Example 4.
[FIG. 13] FIG. 13 is a photograph showing a gel image of the amplified products after the reaction in Example 5.
[FIG. 14] FIG. 14 is a photograph showing a gel image of the amplified products after the reaction in Example 5.
[FIGs. 15A and 15B] FIGs. 15A and 15B are photographs each showing a gel image of the amplified products after the reaction in Example 5.
[FIG. 16] FIG. 16 is a photograph showing a gel image of the amplified products after the reaction in Example 5.
[FIG. 17] FIG. 17 is a photograph showing a gel image of the amplified products after the reaction in Example 5.
[FIG. 18] FIG. 18 is a photograph showing a gel image of the amplified products after the reaction in Example 5.
[FIGs. 19A to 19C] FIGs. 19A to 19C are photographs each showing a gel image of the amplified products after the reaction in Example 6.

### DESCRIPTION OF EMBODIMENTS

The point of the present invention is, for example, that (B) DNA amplifying and (C) RNA amplifying are combined. The inventors of the present invention have found that the amplification efficiency is not so high when only one of (B) DNA amplifying and (C) RNA amplifying is performed, but the reaction efficiency is remarkably improved when (B) DNA amplifying and (C) RNA amplifying are combined. As will be described in Examples, in the amplified products amplified by the amplification method of the present invention, the amount of RNA amplified products are remarkably larger than the amount of DNA amplified products, and therefore it is presumed that a template which is an amplification reaction intermediate (amplicon) is supplied by (B) DNA amplifying, thereby improving the efficiency of (C) RNA amplifying. The present invention, however, is not limited by the above presumption.

The gene amplification method of the present invention may be, for example, an aspect in which the promoter sequence of the RNA polymerase included in the first primer is a T7 promoter sequence, and the RNA polymerase is a T7RNA polymerase.

The gene amplification method of the present invention may be, for example, an aspect in which the RNA genome is a sense strand, the first primer is an antisense primer, and the second primer is a sense primer.

The gene detection method of the present invention may be, for example, an aspect in which, in the amplification, one of the first primer or the second primer is a primer having an antigen capable of specifically binding to an immobilized antibody immobilized on a substrate incorporated at its 5' end, and the other one of the first primer or the second primer is a primer having a first conjugate incorporated at its 5' end, in the detection, the antigen of the one of the first primer or the second primer of a gene amplified product produced in the amplification binds to the immobilized antibody, thereby immobilizing the gene amplified product to the substrate, the first conjugate of the other one of the first primer or the second primer binds to a labeling substance including a second conjugate that specifically binds to the first conjugate via the second conjugate, thereby immobilizing the labeling substance to the substrate, and the labeling substance immobilized on the substrate is detected. Hereinafter, the gene detection method of this aspect may be referred to as, for example, a "first gene detection method of the present invention".

The gene detection method of the present invention may be, for example, an aspect in which the first conjugate is biotin, the second conjugate is streptavidin, and the labeling substance is a colored fine particle having the streptavidin immobilized on its surface.

The gene detection method of the present invention may be, for example, an aspect in which the detection is performed by applying a voltage to a working electrode and a counter electrode and measuring a current between the working electrode and the counter electrode in a presence of a redox substance that binds to a double-stranded site of the amplified product of the target sequence amplified in the amplification. Hereinafter, the gene detection method of this aspect may be referred to as, for example, a "second gene detection method of the present invention".

The gene detection method of the present invention may be, for example, an aspect in which, in the detection, when a current value between the working electrode and the counter electrode is higher than a predetermined reference current value within a specific voltage, a positive determination is made that a target sequence nucleic acid is present in the genome, and when the current value between the working electrode and the counter electrode is lower than the reference current value within a specific voltage, a negative determination is made that the target sequence nucleic acid is not present in the genome.

The gene detection method of the present invention may be, for example, an aspect in which, in the detection, the current between the working electrode and the counter electrode is measured in a presence of a probe nucleic acid capable of complementary binding to the amplified product specifically, and the probe nucleic acid is a probe nucleic acid having a magnetic fine particle with a surface coated with gold bound at its one end and having the redox substance bound at its other end.

The gene detection method of the present invention may be, for example, an aspect in which the redox substance is methylene blue.

As used herein, the term "pathogen" is not particularly limited as long as it has DNA or RNA and is responsible for a disease of an organism. The organism is not particularly limited, and examples thereof include animals (human, domestic animal, and the like), and plants. Specific examples of the pathogen include viruses such as coronaviruses (including SARS-CoV causing SARS, SARS-CoV-2 causing COVID-19, and MERS-CoV causing MERS, for example), influenza virus, norovirus, hepatitis B virus, hepatitis C virus, and the like; bacteria such as *Mycoplasma, Staphylococcus aureus, Streptococcus pneumoniae, Haemophilus influenzae,* ESBL-producing bacteria, *Neisseria gonorrhoeae, Mycobacterium tuberculosis,* hemolytic *Streptococcus, Clostridium difficile,* Enterobacteriaceae, enterococci, *Acinetobacter, Pseudomonas aeruginosa, Staphylococcus aureus, Campylobacter,* nontyphoidal *Salmonella,* Salmonella typhi, *Shigella,* Group *A Streptococcus,* Group B *Streptococcus,* non-tuberculous mycobacteria, spirochetes, and the like; and fungi such as *Trichophyton, Candida,* and the like.

### <Gene amplification method>

The gene amplification method of the present invention is, as described above, an isothermal gene amplification method of a target sequence in an RNA genome, and is characterized in that it includes the following (A) producing an amplification reaction template; (B) DNA amplifying for increasing the amplification reaction template; and (C) RNA amplifying, and other steps and conditions are not particularly limited:
(A) producing an amplification reaction template, including:
   (A1) hybridizing, a first primer having a promoter sequence of an RNA polymerase on its 5' side and a complementary sequence complementary to a 5'-side sequence of the target sequence in the RNA genome on its 3' side, to the 5'-side sequence of the target sequence in the RNA genome, and extending a 3' end of the first primer by reverse transcription activity of a reverse transcriptase to produce a double-stranded complex of the RNA genome and an extended DNA strand;
   (A2) degrading the RNA genome of the double-stranded complex formed in (A1) by RNA degrading activity of the reverse transcriptase to produce a single-stranded DNA having a first primer sequence; and
   (A3) hybridizing a second primer having the same sequence as a 3'-side sequence of the target sequence to a sequence complementary to the target sequence of the single-stranded DNA produced in (A2), and extending a 3' end of the second primer by DNA synthetic activity of the reverse transcriptase to produce a single-stranded DNA having a second primer sequence, thereby producing a double-stranded DNA as the amplification reaction template;
(B) DNA amplifying for increasing the amplification reaction template, including:
   (B 1) hybridizing the second primer to a 3' side of the single-stranded DNA including the first primer and hybridizing the first primer to a 3' side of the single-stranded DNA having the second primer sequence in the double-stranded DNA as the amplification reaction template produced in (A3) by a recombinase in a presence of a single-stranded DNA-binding protein; and
   (B2) extending the 3' end of the first primer and the 3' end of the second primer by strand displacement DNA synthetic activity of a strand displacement DNA polymerase in a presence of the single-stranded DNA-binding protein to produce a double-stranded DNA that is the same as the amplification reaction template produced in (A3); and
(C) RNA amplifying, including:
   (C1) synthesizing a single-stranded RNA by RNA synthetic activity of an RNA polymerase with a sequence that is downstream on a 3'-side relative to the promoter sequence of the double-stranded DNA as the amplification reaction template produced in (A3) being used as a template sequence;
   (C2) hybridizing the second primer to a 5' side of the single-stranded RNA synthesized in (C1), and extending the 3' end of the second primer by the reverse transcription activity of the reverse transcriptase to produce a double-stranded complex of the single-stranded RNA and an extended DNA;
   (C3) degrading the single-stranded RNA of the double-stranded complex produced in (C2) by the RNA degrading activity of the reverse transcriptase to produce a single-stranded DNA; and
   (C4) hybridizing the first primer to a 3' side of the single-stranded DNA produced in (C3), extending the 3' end of the first primer by the DNA synthetic activity of the reverse transcriptase, and extending a 3' end of the single-stranded DNA produced in (C3) with the promoter sequence of the RNA polymerase of the first primer being used as a template to produce a double-stranded DNA that is the same as the amplification reaction template produced in (A3).

(A) producing an amplification reaction template; (B) DNA amplifying for increasing the amplification reaction template; and (C) RNA amplifying will be described below in order.

The RNA genome may be, for example, a sense strand or an antisense strand, and is preferably a sense strand. When the RNA genome is a sense strand, the specificity of gene amplification by the gene amplification method of the present invention can be improved, for example, as will be described in the Examples.

The "isothermal amplification method" generally refers to a method for performing a nucleic acid amplification reaction isothermally. In the gene amplification method of the present invention, the amplification reaction conditions are not particularly limited, and can be, for example, appropriately determined by a person skilled in the art. In the amplification method of the present invention, the reaction temperature is not particularly limited, and for example, it is preferable to set the reaction temperature around or below the melting temperature (Tm) of the primer, and it is preferable to set the stringency level in consideration of the melting temperature (Tm) of the primer. Specifically, the reaction temperature may be, for example, 33°C to 47°C, 35°C to 45°C, or 37°C to 43°C. In the amplification method of the present invention, the reaction time is not particularly limited, and can be, for example, appropriately determined by a person skilled in the art. Specifically, the reaction time may be, for example, 5 minutes to 60 minutes, 10 minutes to 30 minutes, or 10 minutes to 20 minutes. As will be described in Examples, the reaction time is preferably, for example, 20 minutes or less so that the specificity of amplification by the amplification method of the present invention can be improved.

The target sequence is, for example, a sequence to be amplified in an RNA genome of a target pathogen, and is not particularly limited. The length of the target sequence is not particularly limited, and is, for example, 100 to 400-mer.

The first primer is not particularly limited except that it is, for example, a single-stranded polynucleotide and has a promoter sequence of an RNA polymerase on its 5' side and a complementary sequence complementary to a 5'-side sequence of the target sequence in the RNA genome (hereinafter, also referred to as a "target binding sequence") on its 3' side. The first primer is capable of hybridizing to the 5'-side sequence of the target sequence in the RNA genome, for example, under the reaction conditions in the isothermal gene amplification method of the present invention. In the first primer, the length of the target binding sequence is not particularly limited as long as it is capable of hybridizing to the 5'-side sequence of the target sequence, and is, for example, 20 to 35-mer. For example, the first primer is an antisense primer when the RNA genome is a sense strand and is a sense primer when the RNA genome is an antisense strand, and the first primer is preferably an antisense primer. When the RNA genome is a sense strand and the first primer is an antisense primer, the specificity of gene amplification by the gene amplification method of the present invention can be improved, for example, as will be described in the Examples.

As to the first primers, for example, all of the first primers may have a promoter sequence of an RNA polymerase, or some of the first primers may have a promoter sequence of an RNA polymerase. In the latter case, for example, the first primer that does not have the promoter sequence of the RNA polymerase may have a first binding substance in the gene detection method of the present invention (to be described below) incorporated at its 5' end. Specifically, the first primer that does not include the promoter sequence of the RNA polymerase may be, for example, a biotinylated first binding primer having biotin incorporated as the first binding substance. In this case, in the gene detection method of the present invention to be described below, for example, an amplified product can be easily detected, which is preferable.

The base sequence of the target binding sequence of the first primer is not particularly limited, and can be appropriately set according to the sequence of the target nucleic acid. The target binding sequence can be set, for example, by a conventionally known method, and is usually designed to hybridize to the nucleic acid under stringent conditions so that the target sequence in the RNA genome is contained in an amplified product. The "stringent conditions" can be determined depending on, for example, the melting temperature Tm (°C) of the double-stranded chain of the first primer and a complementary strand thereof, the salt concentration of the hybridization solution, and the like. As a specific example, reference can be made to Sambrook et al., "Molecular Cloning: A Laboratory Manual 2nd Ed." [Cold Spring Harbor Laboratory Press (1989)], which is herein incorporated by reference. For example, if hybridization is performed at a temperature slightly lower than the melting temperature of the primer used, the primer can specifically hybridize to a nucleic acid having a target nucleic acid sequence. Such primers can be designed using commercially available primer construction software, for example, Primer3 (manufactured by Whitehead Institute for Biomedical Research). The "stringent conditions" may be, for example, low stringent conditions, medium stringent conditions, or high stringent conditions. The "low stringent conditions" are, for example, as follows: 5 × SSC, 5 × Denhardt's solution, 0.5% SDS, 50% formamide, and 32°C. The "medium stringent conditions" are, for example, as follows: 5 × SSC, 5 × Denhardt's solution, 0.5% SDS, 50% formamide, and 42°C. The "high stringent conditions" are, for example, as follows: 5 × SSC, 5 × Denhardt's solution, 0.5% SDS, 50% formamide, and 50°C. The degree of stringency can be set by a person skilled in the art, for example, by appropriately selecting conditions such as the temperature, the salt concentration, the concentration and length of a probe, the ionic strength, the time, and the like. As the "stringent conditions", for example, the conditions described in Sambrook et al., "Molecular Cloning: A Laboratory Manual 2nd Ed." [Cold Spring Harbor Laboratory Press (1989)] and the like can be employed.

In the present invention, the basic structures of the first primer and the second primer to be described below are not particularly limited, and may be, for example, an oligonucleotide, a modified oligonucleotide, an oligonucleoside, a modified oligonucleoside, a polynucleotide, a modified polynucleotide, a polynucleoside, a modified polynucleoside, a DNA, a modified DNA, an RNA, a modified DNA, or a chimeric molecule thereof, or the basic structures of the first primer and the second primer may have a structure other than these. The nucleic acid may be, for example, one capable of forming a base pair bond, and in the case of a nucleic acid sample or a target nucleic acid sequence, it may function as a template for, for example, complementary strand synthesis. As the first primer and the second primer, the basic structure is preferably, for example, an oligonucleotide, a polynucleotide, a DNA, or a modified product thereof. In the present invention, the "nucleotide" may be, for example, either of a deoxynucleotide and a ribonucleotide, and the "oligonucleotide" and the "polynucleotide" may be composed of, for example, either of a deoxynucleotide and a ribonucleotide, or may include both a deoxynucleotide and a ribonucleotide. In the present invention, the number of constituent bases of the nucleic acid is not particularly limited. The term "nucleic acid" is generally synonymous with the term "polynucleotide". The term "oligonucleotide" generally refers to a polynucleotide, particularly a polynucleotide having a small number of constituent bases. In general, for example, polynucleotides of 2 to 100-mer, and more generally, polynucleotides of about 2 to 50-mer, are referred to as "oligonucleotides", but "oligonucleotides" are not limited by these numerical values. As used herein, the term "polynucleotide" is intended to include, for example, a polynucleotide and an oligonucleotide.

The promoter sequence of the RNA polymerase is a sequence specifically recognized by RNA polymerase, and a promoter sequence of a known RNA polymerase can be used. Specific examples of the promoter sequence of the RNA polymerase include a T7 promoter sequence specifically recognized by a T7RNA polymerase derived from a bacteriophage T7 and a SP6 promoter sequence specifically recognized by a SP6RNA polymerase derived from a bacteriophage SP6. Specifically, the T7 promoter sequence may be, for example, a polynucleotide represented by the base sequence shown in SEQ ID NO: 1 (5'-aattctaatacgactcactatagggaga-3'). Specifically, the SP6 promoter sequence may be, for example, a polynucleotide represented by the base sequence shown in SEQ ID NO: 2 (5'-atttaggtgacactatagaa-3').

The reverse transcriptase is an enzyme having reverse transcription activity and RNA degrading activity, which is a DNA polymerase that transcribes (reverse-transcribes) a single-stranded RNA into a single-stranded DNA. The reverse transcriptase is not particularly limited, and, for example, a known reverse transcriptase can be used. Specific examples of the reverse transcriptase include M-MuLV reverse transcriptase, AMV reverse transcriptase, transcriptor reverse transcriptase, SUPERSCRIPT^{M} transcriptor reverse transcriptase, and Multi Scribe reverse transcriptase.

The second primer is, for example, a single-stranded polynucleotide, and is not particularly limited except that it has the same sequence as the 3'-side sequence of the target sequence. The expression "having the same sequence as the 3'-side sequence of the target sequence" means, for example, having the same base sequence as the continuous base sequence from the 3' end to the 5' side of the target sequence in the RNA genome. The length of the second primer is not particularly limited, and is, for example, 15 to 30-mer, 20 to 30-mer, or 22 to 27-mer. For example, the second primer is a sense primer when the RNA genome is a sense strand and is an antisense primer when the RNA genome is an antisense strand, and the second primer is preferably a sense primer. When the RNA genome is a sense strand and the second primer is a sense primer, the specificity of gene amplification by the gene amplification method of the present invention can be improved, for example, as will be described in the Examples.

The double-stranded DNA which is the amplification reaction template produced in (A3) is, for example, an amplification intermediate in the amplification method of the present invention, and is also referred to as an amplicon or an amplification reaction template.

The recombinase is a protein that catalyzes the recombination reaction between homologous DNA sequences, and, for example, a known recombinase in a common recombinase polymerase amplification (RPA) method can be used. Specific examples of the recombinase include a UvsX recombinase derived from a bacteriophage T4, a Cre recombinase derived from a bacteriophage P1, a Flp recombinase derived from yeast, and an integrase derived from a bacteriophage ϕ31.

The single-stranded DNA-binding protein is a protein that specifically binds to a single-stranded DNA, and is also referred to as a single-stranded DNA-binding protein (SSB). As the single-stranded DNA-binding protein, for example, a known single-stranded DNA-binding protein used in a common recombinase polymerase amplification (RPA) method can be used. Specific examples of the SSB include single-stranded DNA-binding proteins derived from *E*. *coli*, *Drosophila*, and *Xenopus*; a gene 32 protein derived from a bacteriophage T4 (T4 Gene 32 Protein), and these proteins derived from other species.

The strand displacement DNA polymerase is a DNA synthetase capable of synthesizing a complementary strand while dissociating a double-stranded region where the double-stranded region is present in the direction of extension in the process of synthesizing a DNA strand complementary to the template DNA, i.e., a polymerase having a strand displacement ability (strand displacement activity). As the strand displacement DNA polymerase, for example, a known strand displacement DNA polymerase used in a common recombinase polymerase amplification (RPA) method can be used. The strand displacement DNA polymerase may be, for example, stable at room temperature, stable at medium temperature, or heat-resistant. The polymerase having the strand displacement ability may be, for example, a naturally-derived enzyme, an enzyme prepared by genetic engineering or the like, or a mutant enzyme artificially added with a mutation. As such a polymerase, for example, a DNA polymerase is preferred, and a DNA polymerase having substantially no 5'→3' exonuclease activity is more preferable. Specific examples of a DNA polymerase having the strand displacement ability include, for example, thermophilic *Bacillus* bacterium-derived DNA polymerases such as *Bacillus stearothermophilus* (hereinafter, referred to as "B.st"), *Bacillus caldotenax* (hereinafter, referred to as "B.ca"), *Bacillus thermostearophilus,* and the like; a mutant obtained by deleting the 5'→3' exonuclease activity thereof; and a DNA polymerase I derived from *E. coli* or its Klenow fragment. Besides these, examples of a DNA polymerase having the strand displacement ability include, for example, a Vent DNA polymerase, a Vent (Exo-) DNA polymerase, a DeepVent DNA polymerase, a DeepVent (Exo-) DNA polymerase, a Φ29 phage DNA polymerase, a MS-2 phage DNA polymerase, a Z-Taq DNA polymerase, a Pfu DNA polymerase, a Pfu turbo DNA polymerase, a KOD DNA polymerase, a 9°Nm DNA polymerase, a Therminater DNA polymerase, an Aac DNA polymerase, a Gca DNA polymerase, and a Bsm DNA polymerase.

The strand displacement DNA polymerase may be, for example, a DNA polymerase having reverse transcription activity. Examples of such a strand displacement DNA polymerase include, for example, a BcaBEST DNA polymerase and a Bca(exo-) DNA polymerase. According to such a DNA polymerase, for example, a reverse transcription reaction from a whole RNA or mRNA and a DNA polymerase reaction with cDNA being used as a template can be performed with one type of polymerase. It is also usable to combine a DNA polymerase with the aforementioned reverse transcriptase such as a MMLV reverse transcriptase.

In the gene amplification method of the present invention, the RNA polymerase is, for example, a DNA polymerase that catalyzes a transcription reaction where the DNA polymerase reads the base sequence of a DNA template strand to synthesize a complementary RNA, and a known RNA polymerase can be used. Specific examples of the RNA polymerase include, for example, a T7RNA polymerase derived from a bacteriophage T7 and a SP6RNA polymerase derived from a bacteriophage SP6. The T7RNA polymerase exhibits specificity for the T7 promoter sequence.

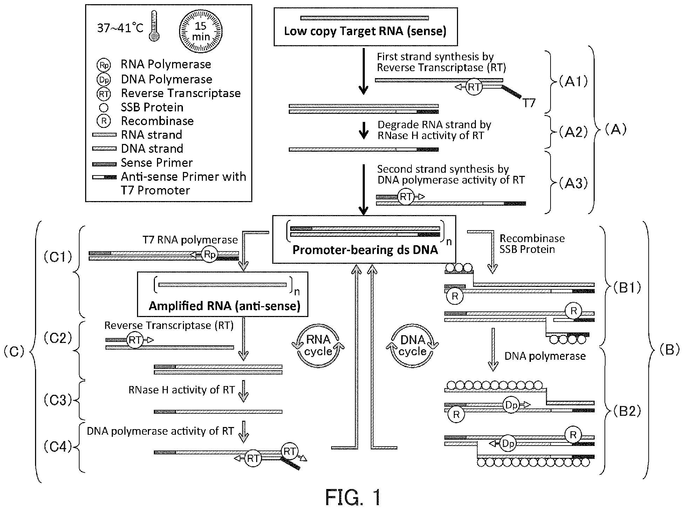

Next, the gene amplification method of the present invention will be described in detail with reference to FIG. 1. FIG. 1 is a schematic diagram showing the mechanism and main steps of the isothermal gene amplification method of the present invention. In the following description, for example, the present invention will be described with reference to a case in which a T7 promoter sequence was used as the promoter sequence of the RNA polymerase and a T7RNA polymerase was used as the RNA polymerase, but the prevent invention is not limited by the following description.

First, as shown in FIG. 1, (A) producing an amplification reaction template is performed. Specifically, a first primer having a T7 promoter sequence on a 5' side is hybridized to a 3' side of the target sequence of a target RNA genome, and a 3' end of the first primer is extended using a reverse transcriptase to produce a double-stranded complex of the RNA genome and an extended DNA strand (A1, reverse-transcription).

Next, the RNA genome of the double-stranded complex is degraded by RNA degrading activity of the reverse transcriptase to produce a single-stranded DNA having the first primer (A2, single-stranded DNA production).

Then, a second primer having the same sequence as a 3'-side sequence of the target sequence is hybridized to a sequence complementary to the target sequence of the single-stranded DNA produced in (A2), and a 3' end of the second primer is extended by DNA synthetic activity of the reverse transcriptase to produce a single-stranded DNA having a second primer sequence, thereby producing a double-stranded DNA as the amplification reaction template (A3, double-stranded DNA production).

Next, (B) DNA amplifying for increasing the amplification reaction template and (C) RNA amplifying shown in FIG. 1 are performed. (B) DNA amplifying and (C) RNA amplifying will be described below in order. As shown in FIG. 1, (B) DNA amplifying and (C) RNA amplifying are performed in parallel and the reactions thereof are proceeded in parallel, for example. Note that the present invention is not limited thereto, and for example, one of (B) DNA amplifying and (C) RNA amplifying may be performed prior to the other.

In (B) DNA amplifying for increasing the amplification reaction template, first, the second primer is hybridized to a 3' side of the single-stranded DNA including the first primer and the first primer is hybridized to a 3' side of the single-stranded DNA having the second primer sequence in the double-stranded DNA as the amplification reaction template produced in (A3) by a recombinase in a presence of a single-stranded DNA-binding protein (B 1, primer hybridization).

Next, the 3' end of the first primer and the 3' end of the second primer are extended by strand displacement DNA synthetic activity of a strand displacement DNA polymerase in a presence of the single-stranded DNA-binding protein to produce a double-stranded DNA that is the same as the amplification reaction template produced in (A3) (B2, double-stranded DNA production). In the present invention, "synthesizing a double-stranded DNA the same as the amplification reaction template" means that the double-stranded DNA produced in (B2) and the amplification reaction template produced in (A3) are substantially the same double-stranded DNAs. The expression "substantially the same" means that, for example, the sense strand and the antisense strand of the double-stranded DNA produced in (B2) can hybridize to the antisense strand and the sense strand of the amplification reaction template produced in (A3), and the sense strand and the antisense strand of the amplification reaction template produced in (A3) can hybridize to the antisense strand and the sense strand of the double-stranded DNA produced in (B2). Specifically, the double-stranded DNA produced in (B2) and the amplification reaction template produced in (A3) may be completely the same sequences (full match) or sequences (mismatch) in which some bases are different from each other. As to the mismatch, for example, one of the two sequences may be a sequence obtained by at least one of deleting, substituting, and inserting a base(s) of the other sequence. The number of bases deleted from, substituted with, or inserted into the sequence is preferably less than 10% of the sum of the number of bases in the double-stranded DNA and amplification reaction template. Particularly preferably, the double-stranded DNA and the amplification reaction template are completely the same, i.e. no deletion, substitution or insertion has occurred. In addition, the C-terminus of the sequence is excluded, for example, for a site where deletion, substitution or insertion occurs.

Thereafter, by repeating (B1) and (B2) using the double-stranded DNA the same as the amplification reaction template produced in (B2) until the reaction stops, the double-stranded DNAs the same as the amplification reaction template are produced.

In (C) RNA amplifying, first, a single-stranded RNA is synthesized by RNA synthetic activity of a T7 RNA polymerase with a sequence at 3'-side downstream relative to the T7 promoter of the double-stranded DNA as the amplification reaction template produced in (A3) being used as a template (C1, single-stranded RNA synthesizing).

Next, the second primer is hybridized to a 5' side of the single-stranded RNA synthesized in (C1), and the 3' end of the second primer is extended by the reverse transcription activity of the reverse transcriptase to produce a double-stranded complex of the single-stranded RNA and an extended DNA (C2, reverse-transcription).

Next, the single-stranded RNA of the double-stranded complex produced in (C2) is degraded by the RNA degrading activity of the reverse transcriptase to produce a single-stranded DNA (C3, single-stranded DNA production).

Then, the first primer is hybridized to a 3' side of the single-stranded DNA produced in (C3), the 3' end of the first primer is extended by the DNA synthetic activity of the reverse transcriptase, and a 3' end of the single-stranded DNA produced in (C3) is extended with a T7 promoter sequence of the first primer being used as a template to produce a double-stranded DNA that is the same as the amplification reaction template produced in (A3) (C4, double-stranded DNA production). In the present invention, "synthesizing a double-stranded DNA that is the same as the amplification reaction template" means that the double-stranded DNA produced in (C4) and the amplification reaction template produced in (A3) are substantially the same double-stranded DNAs. The expression "substantially the same" means that, for example, the sense strand and the antisense strand of the double-stranded DNA produced in (C4) can hybridize to the antisense strand and the sense strand of the amplification reaction template produced in (A3), and the sense strand and the antisense strand of the amplification reaction template produced in (A3) can hybridize to the antisense strand and the sense strand of the double-stranded DNA produced in (C4). Specifically, the double-stranded DNA produced in (C4) and the amplification reaction template produced in (A3) may be completely the same sequences (full match) or sequences (mismatch) in which some bases are different from each other. As to the mismatch, for example, one of the two sequences may be a sequence obtained by at least one of deleting, substituting, and inserting a base(s) of the other sequence. The number of bases deleted from, substituted with, or inserted into the sequence is preferably less than 10% of the sum of the number of bases in the double-stranded DNA and an amplification reaction template. Particularly preferably, the double-stranded DNA and the amplification reaction template are completely the same, i.e., no deletion, substitution or insertion has occurred. In addition, the C-terminus of the sequence is excluded, for example, for a site where deletion, substitution or insertion occurs.

Thereafter, by repeating (C1) to (C4) using the double-stranded DNA that is the same as the amplification reaction template produced in (C4) until the reaction stops, the single-stranded RNAs and the double-stranded DNAs that are the same as the amplification reaction template, are produced.

As described above, the isothermal gene amplification method of the present invention is characterized in that (B) DNA amplifying and (C) RNA amplifying are combined. Thus, for example, when (B) DNA amplifying and (C) RNA amplifying are performed in parallel, it is presumed that a template which is an amplification reaction intermediate (amplicon) is supplied by (B) DNA amplifying, thereby improving the efficiency of (C) RNA amplifying. The present invention, however, is not limited by the above presumption.

The isothermal gene amplification method of the present invention may include, for example, extracting the RNA genome from a sample prior to (A), (B), and (C). The sample may be, for example, a biological sample collected from a subject or an environmental sample collected from an environment.

In the present invention, the sample (also referred to as a specimen) is not particularly limited, and examples thereof include biological samples such as saliva, nasal aspirate, nasal wash, nasal swab, nasal discharge, pharyngeal swab, pharyngeal solution, whole blood, serum, plasma, sweat, urine, and the like, and the sample is preferably a viscous specimen. The viscous specimen is not particularly limited, and examples thereof include saliva, nasal aspirate, nasal wash, nasal swab, nasal discharge, pharyngeal swab, and pharyngeal solution. The specimen is not limited to a liquid specimen, and may be a solid specimen dissolved in a buffer solution or the like, and examples of the solid specimen include biological samples such as cells and feces; food such as animals and plants; and food product such as processed food. The buffer solution is not particularly limited, and may be, for example, the buffer solution described above or the like.

In the RNA extracting, the method for extracting RNA is not limited in any way, and conventionally known methods such as guanidine isothiocyanate and phenol-chloroform extraction can be employed. A commercially available RNA extraction reagent may also be used.

### <First kit>

The kit (first kit) for use in the isothermal gene amplification method of the present invention, as described above, includes:
(a1) the reverse transcriptase;
(a2) the first primer;
(a3) the second primer;
(b 1) the recombinase;
(b2) the single-stranded DNA-binding protein;
(b3) the strand displacement DNA polymerase; and
(c1) the RNA polymerase.
According to the first kit of the present invention, the isothermal gene amplification method of the present invention can be easily and simply performed.

The first kit of the present invention is characterized in that it includes (a1) to (a3), (b 1) to (b3) and (c1), and the configuration and conditions are not particularly limited. In the first kit of the present invention, the reverse transcriptase, the first primer, the second primer, the recombinase, the single-stranded DNA-binding protein, the strand displacement DNA polymerase, and the RNA polymerase are not particularly limited, and reference can be made to the description as to the isothermal gene amplification method of the present invention.

The first kit of the present invention may include components other than (a1) to (a3), (b 1) to (b3), and (c1). Other component may be, for example, an instruction manual. In addition, the first kit of the present invention may further include, for example, substrates such as a dNTP mix (dATP, dTTP, dCTP, dGTP) and the like; buffers such as a Tris HCL buffer, a Tricin buffer, a sodium phosphate buffer, a potassium phosphate buffer, and the like; catalysts such as magnesium chloride, magnesium acetate, magnesium sulfate, and the like; additives such as dimethyl sulfoxide (DMSO), betaine (N,N,N-trimethylglycine), and the like; cationic complexes; and enzyme stabilizers. The enzyme stabilizer is not particularly limited, and examples thereof include glycerol, bovine serum albumin, and saccharides, and among them, preferred are saccharides, more preferred are monosaccharides or oligosaccharides, and still more preferred is trehalose, sorbitol or mannitol, or a mixture of two or more of these. The first kit of the present invention may further include a melting temperature adjuster. Examples of the melting temperature adjuster include DMSO, betaine, formamide or glycerol, or any combination thereof, and preferably DMSO. In the first kit of the present invention, the ratio and the like of these reagents are not particularly limited, and can be appropriately determined by a person skilled in the art. The first kit of the present invention may include, for example, a commercially available reagent. Specifically, the commercially available reagent is, for example, a Twist Amp^{®} Basic kit (TwistDx Inc.).

In the first kit of the present invention, the reagents such as (a1) to (a3), (b 1) to (b3) and (c1) and the other components may be contained in separate containers or may be contained in the same container. The first kit of the present invention can also be referred to, for example, as an isothermal gene amplification reagent for use in the isothermal gene amplification method of the present invention.

When the isothermal gene amplification method of the present invention includes the RNA extracting, the first kit of the present invention may further include, for example, a commercially available RNA extraction reagent.

### <Gene detection method>

As described above, the gene detection method of the present invention is characterized in that it is a gene detection method for detecting a gene of the target sequence of the RNA genome, including amplifying a gene and detecting an amplified gene, wherein the amplification is performed by the gene amplification method of the present invention, and the other steps and conditions are not limited in any way. Regarding the gene detection method of the present invention, reference can be made, for example, to the description as to the gene amplification method and the first kit of the present invention.

In the gene detection method of the present invention, the detecting is not particularly limited, and for example, a known amplified gene detection method such as agarose gel electrophoresis can be used, but it is preferable that the detection is performed by the first gene detection method or the second gene detection method of the present invention to be described below. A specific example of the detection will be described below. In the gene detection method of the present invention, however, the detection is not limited to the implementation of the gene detection method in the following examples.

### <First gene detection method>

A specific example of the first gene detection method of the present invention will be described. In the first gene detection method, for example, in the amplification, one of the first primer or the second primer is a primer having an antigen capable of specifically binding to an immobilized antibody immobilized on a substrate incorporated at its 5' end, and the other of the first primer and the second primer is a primer having a first conjugate incorporated at its 5' end, in the detecting, the antigen of the one of the first primer or the second primer of a gene amplified product produced in the amplification binds to the immobilized antibody, thereby immobilizing the gene amplified product to the substrate, the first conjugate of the other primer binds to a labeling substance including a second conjugate that specifically binds to the first conjugate via the second conjugate, thereby immobilizing the labeling substance to the substrate, and the labeling substance immobilized on the substrate is detected.

In the first gene detection method of the present invention, regarding the detection, reference can be made, for example, to various steps, conditions, reagents, and the like in a common lateral flow immunoassay (LFIA). Specifically, the first gene detection method of the present invention can be performed by, for example, the method described in the Examples to be described below.

The antigen incorporated in either of the first primer and the second primer on its 5' side is not particularly limited as long as it is a known antigen capable of forming an antigen-antibody complex with the immobilized antibody to be described below. Specifically, the antigen may be, for example, digoxin. In the following description, among the first primer and the second primer, the primer in which the antigen is incorporated is also referred to as, for example, a labeled primer.

The binding between the antigen and the 5' side of either of the first primer and the second primer may be, for example, direct binding or indirect binding. The indirect binding may be, for example, binding via a linker.

The immobilized antibody is not particularly limited as long as it is capable of binding to an antigen bound to the first primer or the second primer, i.e., the immobilized antibody is not particularly limited as long as it is capable of forming the antigen-antibody complex. When the antigen is, for example, the digoxin, the antibody may be an anti-digoxin antibody. The method for immobilizing the immobilized antibody to the substrate is not particularly limited, and for example, a conventionally known method can be used, such as applying an antibody solution containing the immobilized antibody on the porous material of the substrate using an application device or the like, and air-drying it using a dryer or the like.

The first conjugate is not particularly limited as long as it is capable of specifically binding to the second conjugate to be described below, and examples thereof include biotin and biotin analogs. The biotin analog may be, for example, desthiobiotin or the like. In the following description, when the first conjugate is biotin, among the first primer and the second primer, the primer in which the first conjugate is incorporated is also referred to as a biotinylated primer.

The binding between the first conjugate and the 5' side of either of the first primer and the second primer may be, for example, direct binding or indirect binding. The indirect binding may be, for example, binding via a linker.

The second conjugate is not particularly limited as long as it is capable of specifically binding to the first conjugate, and when the first conjugate is biotin, for example, the second conjugate may be avidin or an avidin analog. Examples of the avidin analog include streptavidin and neutravidin.

The labeling substance is, for example, a colored fine particle having the second conjugate immobilized on its surface. The colored fine particle is not particularly limited, and examples thereof include colored latex particles, metal colloid particles, colored polymethyl methacrylate particles, colored polylactic acid particles, colored porous glass particles, colored silica particles, colored agarose particles, and colored dextran particles. The colored latex particle is not particularly limited, and examples thereof include blue latex particles and red latex particles. The metal colloidal particle is not particularly limited, and examples thereof include gold colloidal particles and platinum colloidal particles. The mean particle size of the colored insoluble carrier particles is not particularly limited. In the case of the colored latex particles, the mean particle size is, for example, in the range from 0.05 µm to 5 µm, and preferably in the range from 0.1 µm to 1 µm. In the case of the metal colloid particles, the mean particle size is, for example, in the range from 2 nm to 100 nm, and preferably in the range from 10 nm to 50 nm.

As the substrate, for example, a substrate used in a known lateral flow immunoassay can be used. The substrate includes, for example, a support, a porous membrane which is a porous material, a conjugate pad, and a sample pad, wherein the porous membrane having a length shorter than that of the support is disposed on the support, the conjugate pad is disposed on one end of the surface of the porous membrane, and the sample pad is disposed on other end of the conjugate pad on the side opposite to the side where the porous membrane is disposed. The sample pad is a specimen supply site to which a specimen containing the gene amplified product produced in the amplification is supplied, and the sample pad side is the upstream side of the flow of the specimen. On the surface of the porous membrane, detection sites on each of which the immobilized antibody is immobilized are formed in order from the upstream side. An absorption pad is disposed, for example, on the end of the surface of the porous membrane on the side opposite to the side where the conjugate pad is disposed. The conjugate pad may be placed on the porous membrane in its entirety or in part. The sample pad may be placed on the conjugate pad in its entirety or in part.

The material of the support is not particularly limited, and examples thereof include polyethylene terephthalate, polyethylene, polystyrene, polyester, and cellulose acetate. The shape of the support is not particularly limited, and examples thereof include a film shape, a sheet shape, and a plate shape. The shape and size of the support are not particularly limited, and may be appropriately set according to other configurations and the like.

The porous membrane is not particularly limited as long as it exhibits a capillary action, and examples thereof include a cellulose membrane; a cellulose derivative membrane such as a cellulose acetate membrane, a nitrocellulose membrane, or the like; a glass filter; and a filter paper. In the present invention, the shape of the porous membrane is not particularly limited, and examples thereof include a rectangular shape and a circler shape. In the present invention, the size of the porous membrane is not particularly limited, and can be appropriately set. In the present invention, the porous material is not limited to the porous membrane, and examples thereof include particulate matters and particulate powder, such as polymer beads, glass beads, titanium dioxide, cellulose, salts, hydrophobized polysaccharides, and the like. The porous material is not particularly limited as long as it has a porous structure. In the present invention, the shape and size of the porous material are not particularly limited, and can be appropriately set. The porous membrane may be, for example, disposed on the support by a conventional method. Specifically, the porous membrane may be immobilized on the support using a double-sided tape or an adhesive.

The material of the conjugate pad is not particularly limited, and examples thereof include polyethylene, glass fiber, rayon, nylon, paper, and cellulose. The shape and size of the conjugate pad are not particularly limited, and can be appropriately set. The conjugate pad holds, for example, the labeling substance containing the second conjugate.

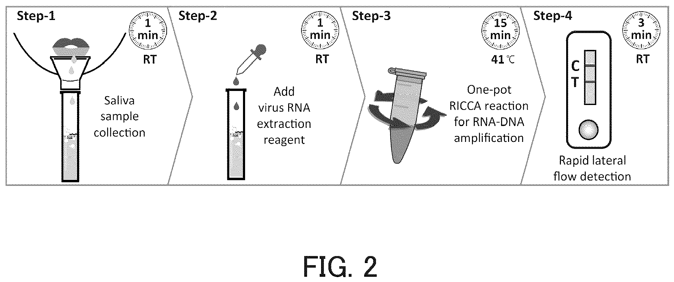

A specific example of the first gene detection method of the present invention will be described with reference to FIG. 2. FIG. 2 is a schematic diagram showing main steps of extracting RNA from a saliva sample of a subject and detecting a gene by the first gene detection method of the present invention.

First, a saliva sample collection tube (e.g., SalivaBio Oral Swab (trade name), Salimetrics, LLC) is used to collect a saliva sample of a subject (Step-1). Next, a commercially available RNA extraction reagent (e.g., a NP40 detergent-containing buffer) is added to the saliva sample and mixed, and the viral particles are dissolved therein to extract RNA (Step-2). Next, the dissolved sample and the first kit of the present invention are mixed to perform the amplification. The amplification can be performed, for example, at 41°C for 15 minutes. Then, the sample containing the amplified product produced in the amplification is subjected to, for example, a substrate (also referred to as a lateral flow dipstick (LFD)) on which an antibody that specifically binds to the antigen bound to the first or second primer is immobilized. When the target sequence of the target genomic RNA is contained in the saliva sample, the antigen of the labeled primer in the amplified product binds to the immobilized antibody on the substrate. Then, the biotin incorporated in the biotinylated primer in the amplified product reacts with the streptavidin-binding labeling substance in the LFD, and the detection site develops color by the labeling substance. Therefore, it is possible to determine the presence of a target sequence in the sample based on whether or not the detection site develops color, that is, it is possible to detect a target gene in the sample.

### <Kit for use in first gene detection method> (Second kit)

The kit (second kit) for use in the first gene detection method of the present invention, as described above, includes:
(a1) the reverse transcriptase;
(a2) the first primer;
(a3) the second primer;
(b1) the recombinase;
(b2) the single-stranded DNA-binding protein;
(b3) the strand displacement DNA polymerase;
(c1) the RNApolymerase;
(d1) the immobilized antibody;
(d2) a substrate; and
(d3) a labeling substance.
According to the second kit of the present invention, for example, the first gene detection method of the present invention can be simply performed.

The second kit of the present invention is characterized in that it includes (a1) to (a3), (b1) to (b3), (c1), and (d1) to (d3), and other configurations and conditions are not particularly limited. In the second kit of the present invention, the reverse transcriptase, the first primer, the second primer, the recombinase, the single-stranded DNA-binding protein, the strand displacement DNA polymerase, RNA polymerase, the immobilized antibody, the substrate, and the labeling substance are not particularly limited, and reference can be made to the description as to the isothermal gene amplification method, the first kit, and the first gene detection method of the present invention. The second kit of the present invention is also referred to, for example, as a gene detection reagent for use in the gene detection method of the present invention.

### (Second gene detection method)

In the second gene detection method of the present invention, for example, in the detection, when a current value between the working electrode and the counter electrode is higher than a predetermined reference current value within a specific voltage, a positive determination is made that a target sequence nucleic acid is present in the genome, and when the current value between the working electrode and the counter electrode is lower than the reference current value within a specific voltage, a negative determination is made that the target sequence nucleic acid is not present in the genome.

The second gene detection method of the present invention may be an aspect in which, in the detection process, the current between the working electrode and the counter electrode is measured in a presence of a probe nucleic acid capable of complementary binding to the amplified product specifically, and the probe nucleic acid is a probe nucleic acid having a magnetic fine particle with a surface coated with gold bound at one end and having the redox substance bound at the other end.

In the second gene detection method of the present invention, the redox substance is, for example, methylene blue.

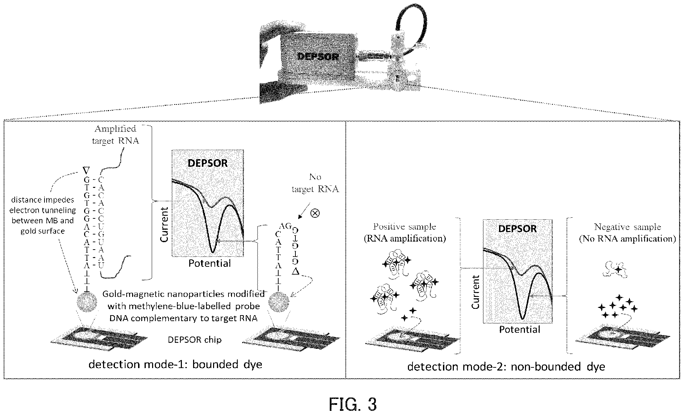

FIG. 3 shows the principle of the detection in the second gene detection method. In FIG. 3, a disposable electrochemical print sensor (DEPSOR) is a technology developed by the inventors of the present invention. FIG. 3 shows two modes of detection. One mode is a mode using a probe nucleic acid, wherein a magnetic nanoparticle coated with gold (AuMNP) is bound to one end of the probe nucleic acid and a redox substance (methylene blue, MB) is bound to the other end of the probe nucleic acid. The probe nucleic acid has a complementary sequence that specifically binds to an amplified product produced in the amplification. The electron tunneling effect due to the interaction of gold and a redox substance can be adjusted by the length of the probe nucleic acid. When the voltage is applied to the working electrode and the counter electrode in the presence of the probe nucleic acid, the value of the current flowing between the working electrode and the counter electrode changes depending on the presence or absence of the target sequence of the amplified product, and thus the presence or absence (positive or negative) of the target sequence can be determined by this change. In another mode, when the voltage is applied to the working electrode and the counter electrode in the presence of the redox substance (MB), the value of the current flowing between the working electrode and the counter electrode changes depending on whether or not the redox substance binds to a complementary double-stranded moiety formed in the amplified product (e.g., whether or not intercalation occurs), and thus the presence or absence of the target sequence (positive, negative) can be determined by this change.

### <Kit for use in second gene detection method> (Third kit)

The kit (hereinafter also referred to as "third kit") for use in the second gene detection method of the present invention, as described above, includes:
(a1) the reverse transcriptase;
(a2) the first primer;
(a3) the second primer;
(b 1) the recombinase;
(b2) the single-stranded DNA-binding protein;
(b3) the strand displacement DNA polymerase;
(c1) the RNApolymerase;
(d1) a working electrode and a counter electrode; and
(d2) a redox substance or a nucleic acid probe that binds to the double-stranded site of the amplified product.
According to the third kit of the present invention, for example, the second gene detection method of the present invention can be simply performed.

The third kit of the present invention is characterized in that it includes (a1) to (a3), (b 1) to (b3), (c1), (d1), and (d2), and other configurations and conditions are not particularly limited. In the third kit of the present invention, the reverse transcriptase, the first primer, the second primer, the recombinase, the single-stranded DNA-binding protein, the strand displacement DNA polymerase, RNA polymerase, the working electrode and the counter electrode, and the redox substance or the nucleic acid probe that binds to the double-stranded site of the amplified product are not particularly limited, and reference can be made to the description as to the isothermal gene amplification method, the first kit, and the second gene detection method of the present invention. The third kit of the present invention is also referred to, for example, as a gene detection reagent for use in the gene detection method of the present invention.

### <Virus detection method >

The virus detection method of the present invention is an RNA virus detection method. The RNA virus detection method is a method for detecting a sequence specific to the RNA virus in a viral genome as a target sequence, wherein the detection of the target sequence is performed by the gene detection method of the present invention.

The virus detection method of the present invention is characterized in that the detection of the target sequence is performed by the gene detection method of the present invention, and for example, other steps and conditions are not particularly limited. Regarding the detection of the target sequence in the virus detection method of the present invention, reference can be made, for example, to the description as to the isothermal gene amplification method and the gene detection method of the present invention.

### <Virus detection kit>

The virus detection kit of the present invention is a detection kit for use in the virus detection method of the present invention. The virus detection kit of the present invention is characterized in that it includes the second kit or the third kit of the present invention, and other configurations are not particularly limited. Regarding the virus detection kit of the present invention, reference can be made, for example, to the description as to the isothermal gene amplification method, the first kit, the first and second gene detection methods, the second kit, the third kit, and the virus detection method of the present invention. The virus detection kit of the present invention is also referred to as a virus detection reagent, for example, for use in the virus detection method of the present invention.

### Examples

Next, examples of the present invention will be described. It is to be noted, however, that the present invention is not restricted by the following examples. Commercially available reagents were used according to their protocol unless otherwise indicated.

### Example 1

It was examined that a target sequence can be amplified by the amplification method of the present invention.

### (1) Preparation of RNA genomic samples

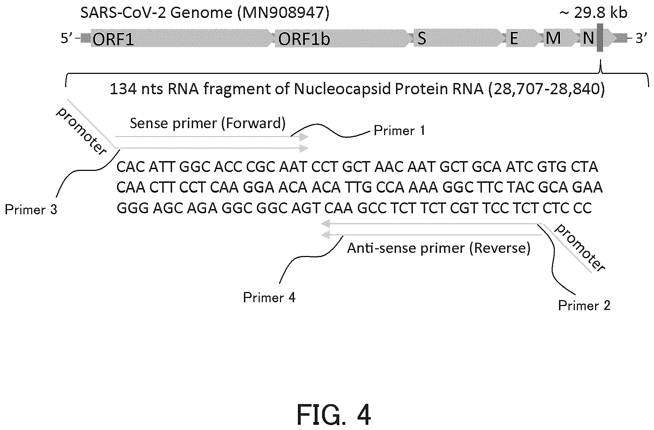

The sequence of the nucleocapsid gene (N gene) of SARS-CoV-2 was identified from GenBank (Accession Number Mn908947) and a single-stranded DNA fragment at 134 nts corresponding to the RNA sequence at positions 28707 to 28840 was synthesized *in vitro* by a conventional method. A T7 RNA polymerase promoter sequence was added to the end of the DNA fragment, and the resultant was subjected to overlap extension PCR using DNA oligomers (primers 1 to 3 for overlap extension, SEQ ID NOs: 3 to 5, purchased from Eurofins Genomics K.K., Tokyo, Japan) below, thereby synthesizing a double-stranded DNA construct. From the double-stranded DNA construct, an RNA transcript was synthesized using a cell-free transcription kit (RIBOMAX^{™}, Promega Corporation) and purified using a commercially available RNA purification kit (RNeasy, QIAGEN). The concentration of the purified RNA transcript was measured using a spectrophotometer (NanoDrop^{™} ND-2000, Thermo Fisher Scientific Inc., Waltham, MA) and a standard template from 10⁹ to 1 RNA copies/µl was serially diluted with a TE buffer, thereby preparing an RNA genomic sample (406 nts) of the sense and antisense strands of SARS-CoV-2. The target sequence (SEQ ID NO: 6) in the prepared RNA genomic sample is shown in FIG. 4.
Primer 1 for overlap extension (forward primer, SEQ ID NO: 3) 
5'- aaaatgaaag atctcagtcc aagatggtat ttctactacc taggaactgg gccagaagct ggacttccct atggtgctaa
caaagacggc atcatatggg ttgcaactga gggagccttg -3'
Primer 2 for overlap extension (forward primer, SEQ ID NO: 4) 
5'- ttgcaactga gggagccttg aatacaccaa aagatcacat tggcacccgc aatcctgcta acaatgctgc aatcgtgcta
caacttcctc aaggaacaac attgccaaaa ggcttctacg cagaagggag cagaggcggc agtcaagcct cttctcgttc
ctcatcacgt agtcgcaaca gttcaagaaa ttcaactcca -3'
Primer 3 for overlap extension (reverse primer, SEQ ID NO: 5)

5'- tgctctcaag ctggttcaat ctgtcaagca gcagcaaagc aagagcagca tcaccgccat tgccagccat tctagcagga
gaagttcccc tactgctgcc tggagttgaa tttcttgaac -3'

FIG. 4 is a schematic diagram showing the position of the target sequence (SEQ ID NO: 6, 134 nts) in the RNA genome of SARS-CoV-2 in the SARS-CoV-2 genome and the positions at which the target sequence hybridizes with the primers of the primer sets 1 and 2 below in the present example. In the base sequences of the primers below, the underlined sequences are T7 promoter sequences.
Target sequence (SEQ ID NO: 6, 134 nts) in RNA genome of SARS-CoV-2 
cacattggcacccgcaatcctgctaacaatgctgcaatcgtgctacaacttcctcaaggaacaacattgccaaaaggcttctacgcagaagg
gagcagaggcggcagtcaagcctcttctcgttcctctctccc
Primer set 1:
   Forward primer 1 (SEQ ID NO: 7)
      5'-CACATTGGCA CCCGCAATC-3'
   Reverse primer 2 (SEQ ID NO: 8)
      5'-AATTCTAATACGACTCACTATAGGGAGAGAGGAACGAGAAGAGGCTTG-3'
Primer set 2:
   Forward primer 3 (SEQ ID NO: 9)
      5'-AATTCTAATACGACTCACTATAGGGAGACACATTGGCACCCGCAATC-3'
   Reverse primer 4 (SEQ ID NO: 10)
      5'-GAGGAACGAGAAGAGGCTTG-3'

### (2) (A) DNA amplifying for increasing amplification reaction template

First, the sense RNA genomic sample of SARS-CoV-2 prepared in (1) was serially diluted such that the amount of the genomic sample became 20 ng (0.87 × 10¹¹), 20 pg (0.87 × 10⁸), and 20 fg (0.87 × 10⁵), thereby preparing genomic samples. Next, 5.0 µl of a substrate stock solution, 5 µl of a primer stock solution, and 2.5 µl of the RNA genomic sample having each of the concentrations were mixed in a PCR tube, incubated at 65°C for 5 minutes, and then incubated at 41°C for another 5 minutes to prepare a reaction mixture. The substrate stock solution was composed of 17.6 µl of nuclease free water (NFW), 5.6 µl of 1 M MgCl₂, 19.8 µl of 1.9 M Tris HCl (pH8.6), 0.6 µl of 40 U/µl RNase Inhibitor, 3.3 µl of 100 mM DTT, 41.3 µl of 2.0 mM dNTP, 17.1 µl of 58 mM NTP, and 4.8 µl of 250 mM ITP. The primer stock solution was composed of 32.5 µl of NFW, 21.5 µl of 2M KCl, 6.6 µl of 50 µM sense forward primer (the forward primer 1), an antisense reverse primer having 6.6 µl of 50 µM T7 promoter in the 5'-side sequence (the reverse primer 2), and 42.9 µl of dimethyl sulfoxide.

Then, 2.5 µl of an enzyme stock solution was added to the reaction mixture to start the reaction, and the reaction was performed at 41°C for 10 to 30 minutes. The enzyme stock solution was composed of 4.0 µl of 10 mg/ml bovine serum albumin, 4.4 µl of 20,000 unit/ml AMV Reverse Transcriptase (Life Sciences Advanced Technologies, Inc., Petersburg, FL), 11.1 µl of 60% Sorbitol, and 35.5 µl of a T7 RNA polymerase solution (TOYOBO CO., LTD.).

### (3) (B) DNA amplifying and (C) RNA amplifying

(B) DNA amplifying and (C) RNA amplifying were performed using a commercially available reagent (TWISTAMP^{®} Basic kit (TwistDx Inc., Cambridge, MA)). Specifically, each of the resulting amplified products was mixed with 5 µl of a primer mix (10 µM digoxin modified sense/forward primer and 10 µM biotin modified antisense/reverse primer) and 29.5 µl of primer-free replacement buffer. Next, to the mixed solution (49.5 µl) after the mixing, the dried enzyme pellet included in the TWISTAMP^{®} Basic kit was added, and 2.5 µl of 280 mM magnesium acetate was further added to start the reaction, and was allowed to stand at 41°C for 10 minutes. The amplified product was then analyzed with a 1-inch 6% denatured polyacrylamide gel (ref) and SYBR^{™} Gold Nucleic Acid Gel Stain.
Digoxin modified sense/forward primer (SEQ ID NO: 11)
   5'-Digoxigenin-CACATTGGCA CCCGCAATC-3'
Biotin modified antisense/reverse primer (SEQ ID NO: 12)
   5'-Biotin-GAGGAACGAGAAGAGGCTTG-3'

Further, as Comparative Example 1, the amplified product produced in (2) was analyzed with a 1-inch 6% denatured polyacrylamide gel (ref) and SYBR^{™} Gold Nucleic Acid Gel Stain without performing (3) amplifying ((B) DNA amplifying and (C) RNA amplifying in the amplification method of the present invention).

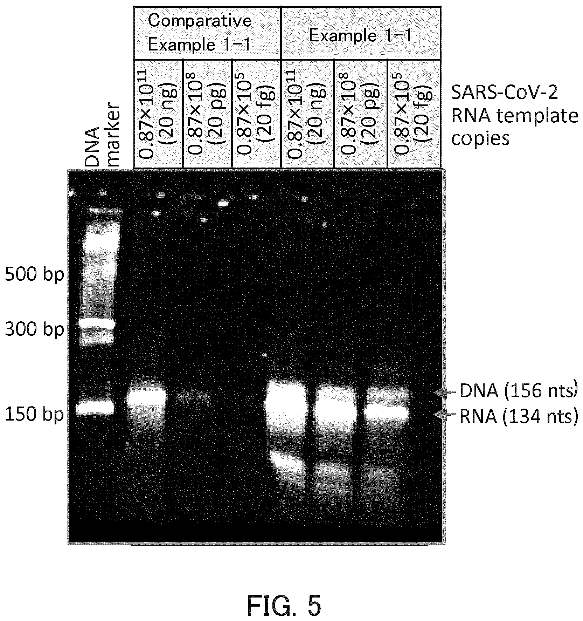

The results are shown in FIG. 5. FIG. 5 is a photograph showing a gel image of the amplified products after the reaction. In FIG. 5, the leftmost lane shows the DNA marker, the second to the fourth lanes from the left show the amplification results of Comparative Example 1-1, and the fifth to the seventh lanes from the left show the amplification results of Example 1-1. In the lanes showing Comparative Example 1-1 and Example 1-1, the lanes in order from the left show the result with 20 ng (0.87 × 10¹¹) of genomic RNA, the result with 20 pg (0.87 × 10⁸) of genomic RNA, and the result with 20 fg (0.87 × 10⁵) of genomic RNA. As shown in FIG. 5, in the amplification results of Comparative Example 1-1, when the amount of the genomic RNA became 20 pg (0.87 × 10⁸) or less, none of the band at the position of 134 nts indicating the RNA amplified product of the target sequence in the genomic RNA and the band at the position of 156 nts indicating the DNA amplified product of the target sequence could be observed, whereas in Example 1-1 using the amplification method of the present invention, both of the band at the position of 134 nts indicating the RNA amplified product of the target sequence in the genomic RNA and the band at the position of 156 nts indicating the DNA amplified product of the target sequence could be observed even with a very small amount of the genomic RNA (20 fg (0.87 × 10⁵). From this, it was found that the gene detection method using the isothermal gene amplification method of the present invention has a very low detection limit (LOD) of the genomic RNA and allows a target sequence to be detected with high sensitivity.

In addition, the amplification method of the present invention was performed in the same manner as described above, except that the amount of the genomic RNA sample was further diluted until the amount of the genomic RNA became 20 ag (87).

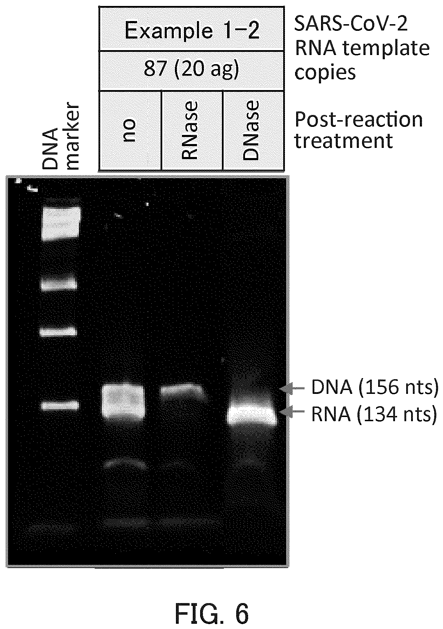

The results are shown in FIG. 6. FIG. 6 is a photograph showing a gel image of the amplified products after the reaction. In FIG. 6, the leftmost lane shows the DNA marker, the second lane from the left shows the result obtained by not being subjected to post-amplification treatment, the third lane from the left shows the result obtained by adding 0.5 U RQ1 RNase-Free DNase (Promega Corporation.) to 5 µl of the amplified product and subjected to DNase treatment at 37 °C for 15 minute, the fourth lane from the left shows the result obtained by adding 2.5 U RNase H (Thermo Fisher Scientific Inc.) and 2.5 U RNase ONE Ribonuclease (Promega Corporation.) to 5 µl of the amplified product and subjected to RNase treatment at 37°C for 20 minutes. As shown in FIG. 6, according to the amplification method of the present invention, even with a very small amount of the genomic RNA (20ag (87)), both of the band at the position of 134 nts indicating the RNA amplified product of the target sequence in the genomic RNA and the band at the position of 156 nts indicating the DNA amplified product of the target sequence were observed. From this, it was found that the gene detection method using the isothermal gene amplification method of the present invention has a very low detection limit (LOD) of the genomic RNA and allows a target sequence to be detected with high sensitivity.

Next, the amount of the genomic RNA was set to 20 ng (0.87 × 10¹¹), and the effect of the presence or absence of the reverse primer 2 (SEQ ID NO: 8) on the amplification method of the present invention was examined.

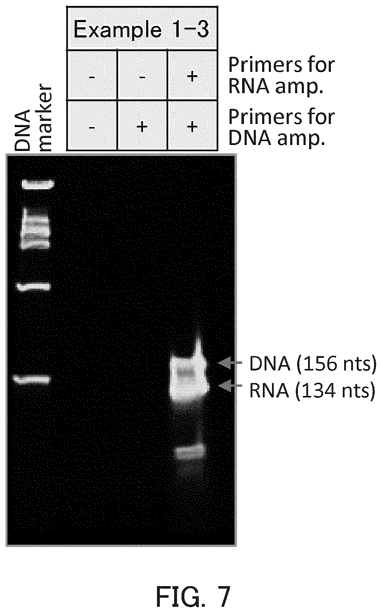

The results are shown in FIG. 7. FIG. 7 is a photograph showing a gel image of the amplified products after the reaction. In FIG. 7, the leftmost lane shows the DNA marker, the second lane from the left shows the result without a primer set (negative control), the third lane from the left shows the result obtained by performing the amplification method of the present invention without the reverse primer 2, and the fourth lane from the left shows the result obtained by performing the amplification method of the present invention with the reverse primer 2. As shown in FIG. 7, when the reverse primer 2 is not used, neither the band at the position of 134 nts indicating the RNA amplified product of the target sequence nor the band at the position of 156 nts indicating the DNA amplified product of the target sequence could be observed, whereas when the reverse primer 2 is used, both of the band at the position of 134 nts indicating the RNA amplified product of the target sequence in the genomic RNA and the band at the position of 156 nts indicating the DNA amplified product of the target sequence could be observed. From this, it was found to be important to use a primer including a T7 promoter sequence in the amplification method of the present invention.

### Example 2

It was examined that the gene can be detected by the isothermal gene amplification method of the present invention even in the presence of a saliva sample.

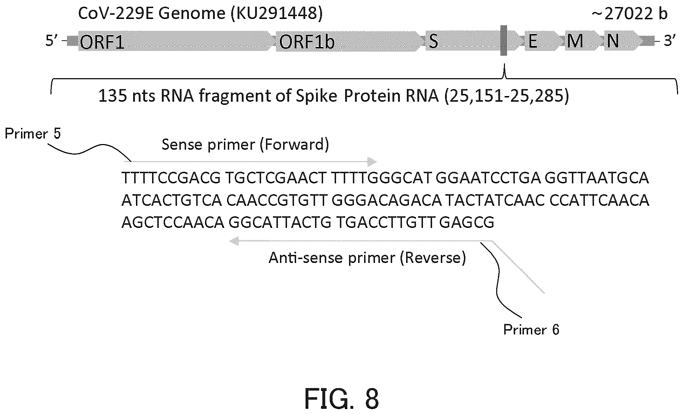

An RNA genomic sample of CoV-229E (135 nts) was prepared in the same manner as in Example 1 (1), except that the sequence of the spike-protein gene (S gene) of CoV-229E, instead of the nucleocapsid gene of SARS-CoV-2, was identified from GenBank (accession number KU291448), and a single-stranded DNA fragment at 135 nts corresponding to the RNA sequence at positions 25151 to 25285 was synthesized *in vitro* by a conventional method, and the DNA oligomers below were used. The target sequence (SEQ ID NO:13) in the prepared RNA genomic sample is shown in FIG. 8.
Primer 4 for overlap extension (forward primer, SEQ ID NO: 14) 
5'- gctaattggg actctaattg ggcctttgtt gcatttagct tccttatggc cgtatcaaca ctcgttatgt gggtgatgta ctttgcaaat
agtttcagac ttttccgacg tgctcgaact ttttgggcat ggaatcctga ggttaatgca atcactgtca -3'
Primer 5 for overlap extension (reverse primer, SEQ ID NO: 15)

5'- gtattcaggt aggttatgaa cctgaacacc tgaagccaat ctatgtccgt caacgtaaag cacgccgctc aacaaggtca
cagtaatgcc tgttggagct tgttgaatgg gttgatagta tgtctgtccc aacacggttg tgacagtgat tgcattaacc -3'

FIG. 8 is a schematic diagram showing the position of the target sequence (SEQ ID NO: 13, 135 nts) in the RNA genome of CoV-229E in the CoV-229E genome and the position at which the target sequence hybridizes with the primer set 3 below in the present example. In the base sequence below, the underlined sequence is a T7 promoter sequence.
Target sequence (SEQ ID NO: 13, 135 nts) in the RNA genome of CoV-229E ttttccgacg tgctcgaact ttttgggcat ggaatcctga ggttaatgca atcactgtca caaccgtgtt gggacagaca tactatcaac ccattcaaca agctccaaca ggcattactg tgaccttgtt gagcg
Primer set 3:
   Forward primer 5 (SEQ ID NO: 16)
      5'-TTTTCCGACGTGCTCGAACTTTTTG-3'
   Reverse primer 6 (SEQ ID NO: 17)
      5'-AATTCTAATACGACTCACTATAGGGAGACGCTCAACAAGGTCACAGTAATGCC-3'

The isothermal gene amplification method of the present invention was performed in the same manner as in Example 1 (2) to (3), except that the RNA genome of CoV-229E of was used instead of the RNA genome of SARS-CoV-2, the primer set 3 was used instead of the primer set 1, and a saliva sample was further added in an amount of 5% with respect to the total weight of the reaction solution (Example 2). The amount of the genomic RNA was 20 pg (0.87 × 10⁸). As Comparative Example 2, the amplified product produced in (2) was analyzed with a 1-inch 6% denatured polyacrylamide gel (ref) and SYBR^{™} Gold Nucleic Acid Gel Stain without performing (3) amplifying ((B) DNA amplifying and (C) RNA amplifying in the amplification method of the present invention).

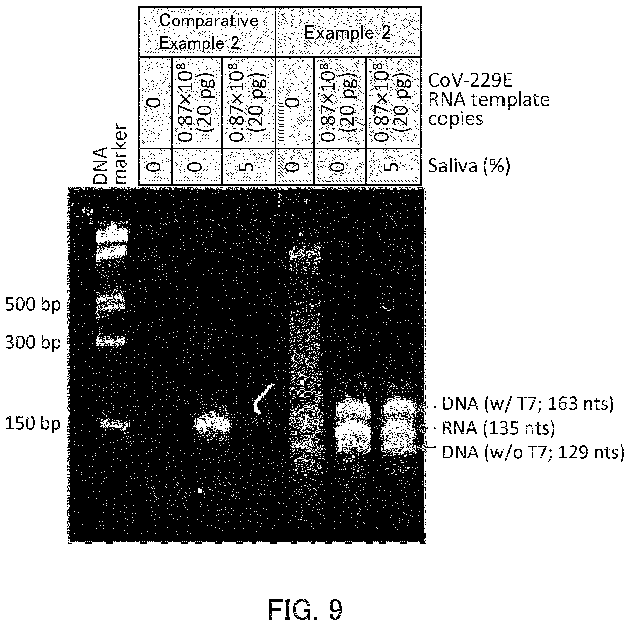

The results are shown in FIG. 9. FIG. 9 is a photograph showing a gel image of the amplified products after the reaction. In FIG. 9, the leftmost lane shows the DNA marker, the second to fourth lanes from the left show the amplification results of Comparative Example 2, and the fifth to seventh lanes from the left show the amplification results of Example 2. In the lanes showing Comparative Example 1 and Example 1, the lanes in order from the left show the result without genomic RNA (negative control), the result without a saliva sample (Comparative Example 2-1, Example 2-1), and the result with a 5% saliva sample (Comparative Example 2-2, Example 2-2). As shown in FIG. 9, in the amplification results of the comparative example, in the presence of the saliva sample, none of the band at the position of 135 nts indicating the RNA amplified product of the target sequence and the bands at the positions of 163 nts and 129 nts indicating the DNA amplified product of the target sequence could be observed, whereas in Example 2 using the amplification method of the present invention, both of the band at the position of 135 nts indicating the RNA amplified product of the target sequence and the bands at the positions of 163 nts and 129 nts indicating the DNA amplified product of the target sequence could be observed even in the presence of the saliva sample. From this, it was found that the gene detection method using the isothermal gene amplification method of the present invention allows a target sequence to be detected regardless of the presence or absence of a saliva sample.

### Example 3

It was examined that the amplified product amplified by the isothermal gene amplification method of the present invention can be detected by the lateral flow immunoassay.

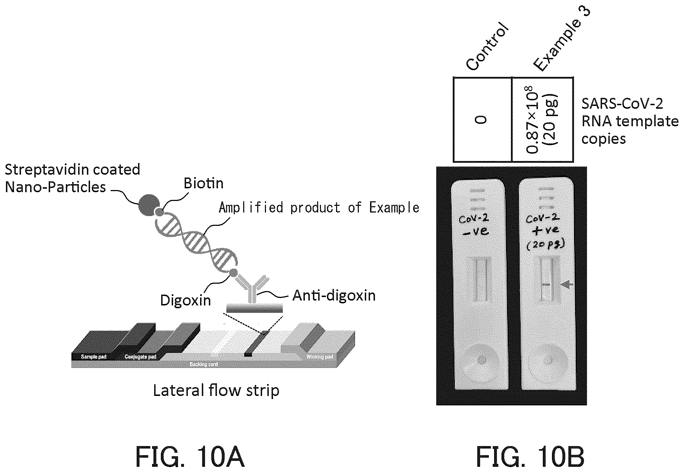

The amplified product of Example 1 was subjected to a lateral flow immunoassay using the lateral flow dipstick (LFD). Details of the LFD are shown in FIG. 10A. As shown in FIG. 10A, the LFD used was prepared by attaching a cover (manufactured by BioSeeds Corporation) having a sample addition site and a detection site to a substrate (lateral flow strip (manufactured by Asahi Kasei Corporation)) including a conjugate pad containing gold colloid-labeled streptavidin and a membrane filter having an anti-digoxin antibody immobilized thereon. Then, to 90 µl of the NFW, 5 µl of the amplified product of 20 pg (0.87 × 10⁸) of a genomic RNA sample of Example 1 or 5 µl of a negative control (an amplified product without a genomic RNA sample) was added, and each of the mixtures was added to a sample pad on the LFD strip. Thereafter, the mixture was allowed to stand for 5 to 10 minutes, and whether or not the detection can be made was determined based on the presence or absence of color development in the detection site of the LFD. The results are shown in FIG. 10.

As shown in FIG. 10B, a detection line indicated by an arrow appeared in the detection site of the LFD to which the amplified product of 20 pg (0.87 × 10⁸) of the genomic RNA sample was provided, and no detection line appeared in the detection site of the LFD to which the amplified product of the negative control was provided. From this, it was found that, according to the isothermal gene amplification method of the present invention, a trace amount of the genomic RNA contained in the sample can be amplified and can be detected by the LF.

### Example 4

It was examined that the process from extraction of RNA from a sample to the isothermal gene amplification method of the present invention can be performed in one container (one-pot reaction).

### (1) Primer design

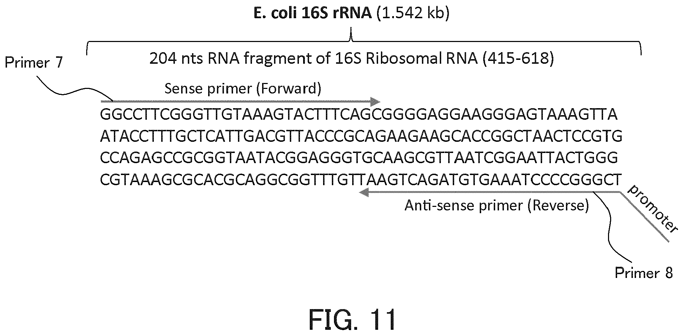

The sequence of *E. coli* 16s rRNA was identified from GenBank (Accession Number J01859.1) and a single-stranded DNA fragment at 204 nts corresponding to the RNA sequence at positions 415 to 618 was synthesized in vitro by a conventional method, thereby producing an RNA genomic sample of 16s rRNA of *E. coli.* The primer sequence shown in FIG. 11 was designed using the RNA sequence at positions 415 to 618 of the 16s rRNA as a target sequence.

FIG. 11 is a schematic diagram showing the positions at which the target sequence (SEQ ID NO: 18, 204 nts) in the RNA genome of 16s rRNA in the present example hybridizes with the primers of the primer set 4. In the base sequence below, the underlined sequence is a T7 promoter sequence.
*E. coli* 16S rRNA target sequence (SEQ ID NO: 18) 
ggccttcgggttgtaaagtactttcagcggggaggaagggagtaaagttaatacctttgctcattgacgttacccgcagaagaagcaccggc
taactccgtgccagagccgcggtaatacggagggtgcaagcgttaatcggaattactgggcgtaaagcgcacgcaggcggtttgttaagtc
agatgtgaaatccccgggct
Primer set 4:
   Forward primer 7 (SEQ ID NO: 19)
      5'-AGCCCGGGGATTTCACATC-3'
   Reverse primer 8 (SEQ ID NO: 20)
      5'-AATTCTAATACGACTCACTATAGGGAGAGGCCTTCGGGTTGTAAAGTAC -3'

### (2) RNA extraction

A commercially available non-pathogenic *E. coli* test strain (NBRC 3301) was obtained from NITE Biological Resource Center, Japan, cultured at 37°C for 24 hours in L broth, and the number of cells (CFU per ml) in a serially diluted bacterial culture was measured using a JuLI-smart fluorescent cell analyzer (Digital Bio, NanoEnTek, Inc., USA) and a hemocytometer (Thermo Fisher Scientific Inc.), and the culture was serially diluted such that the number of cells of *E. coli* became 7.3 × 10⁸ to 8.6 CFU/µl. Cells were then collected from 1 ml of each culture by centrifugation at 15,000 × g for 5 minutes, bacterial cells were disrupted by sonication, and RNA was extracted from the *E. coli* by treatment with a NP40 reagent (SURFACT-AMPS^{™} NP-40, Thermo Fisher Scientific Inc. Thermo Scientific). The solution was then centrifuged at 15,000 × g for 5 minutes to allow the cell debris to settle. The supernatant was used directly as a template for the isothermal amplification reaction and stored at -20°C until use.

### (3) (A) DNA amplifying for increasing amplification reaction template

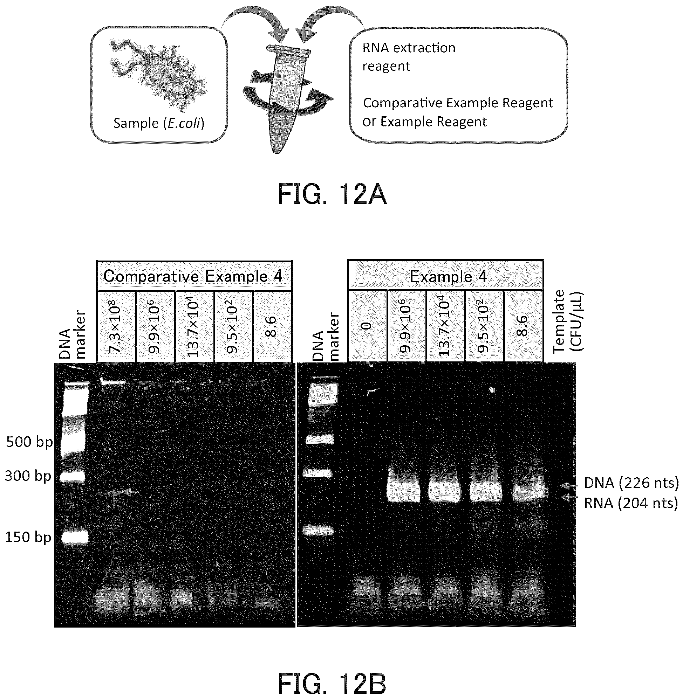

Then, as shown in FIG. 12A, to the supernatant containing RNA extracted from the *E*. *coli,* 5.0 µl of the substrate stock solution used in Example 1 (2), 5 µl of the primer stock solution, and 0.625 µl of human saliva were added, incubated at 65°C for 5 minutes, and then incubated at 41°C for another 5 minutes to prepare a reaction mixture. The composition of the primer stock solution was the same as described above except that the forward primer 7 and the reverse primer 8 were used instead of the forward primer 1 and the reverse primer 2. Then, 2.5 µl of an enzyme stock solution was added to the reaction mixture to start the reaction, and the reaction was performed at 41°C for 10 minutes.

### (4) (B) DNA amplifying and (C) RNA amplifying

The reaction mixture of (A) was mixed with 5 µl of a primer mix (10 µM digoxin modified sense/forward primer and 10 µM biotin modified antisense/reverse primer) and 29.5 µl of primer-free replacement buffer. Next, to the mixed solution (49.5 µl) after the mixing, the dried enzyme pellet included in the TWISTAMP^{®} Basic kit was added, and 2.5 µl of 280 mM magnesium acetate was further added to start the reaction, and was allowed to stand at 41°C for 10 minutes. As Example 4, the amplified product was then analyzed with a 1-inch 6% denatured polyacrylamide gel (ref) and SYBR^{™} Gold Nucleic Acid Gel Stain.
Digoxin modified sense/forward primer (SEQ ID NO: 21)
   5'-Digoxigenin-GGCCTTCGGGTTGTAAAGTACTTTCAGC-3'
Biotin modified antisense/reverse primer (SEQ ID NO: 22)
   5'-Biotin-AGCCCGGGGATTTCACATCTGACTTA-3'

Further, as Comparative Example 4, the amplified product produced in (3) was analyzed with a 1-inch 6% denatured polyacrylamide gel (ref) and SYBR^{™} Gold Nucleic Acid Gel Stain without performing (4) amplification ((B) DNA amplifying and (C) RNA amplifying in the amplification method of the present invention).

The results are shown in FIG. 12B. FIG. 12B is a photograph showing a gel image of the amplified products after the reaction. In FIG. 12B, the gel image on the left is a photograph showing the results of Comparative Example 4, and the gel image on the right is a gel image showing the results of Example 4. In the gel image showing the results of Comparative Example 4, the lanes in order from the left show the DNA marker, the result with the number of *E. coli* cells as a template being 7.3 × 10⁸ CFU cells/µl, the result with the number of *E. coli* cells as a template being 9.9 × 10⁶ CFU cells/µl, the result with the number of *E. coli* cells as a template being 13.7 × 10⁴ CFU cells/µl, the result with the number of *E. coli* cells as a template being 9.5 × 10² CFU cells/µl, and the result with the number of *E. coli* cells as a template being 8.6 CFU/µl. Further, in the gel image showing the results of Example 4, the lanes in order from the left show the DNA marker, the result without *E. coli* (negative control), the result with the number of *E. coli* cells as a template being 9.9 × 10⁶ CFU ells/µl, the result with the number of *E. coli* cells as a template being 13.7 × 10⁴ CFU ells/µl, the result with the number of *E. coli* cells as a template being 9.5 × 10² CFU ells/µl, and the result with the number of *E. coli* cells as a template being 8.6 CFU/µl. As shown in FIG. 12B, in the amplification results of Comparative Example, in the presence of saliva, none of the band at the position of 204 nts indicating the RNA amplified product of the target sequence and the band at the position of 226 nts indicating the DNA amplified product of the target sequence could be observed, whereas in Example 4 using the amplification method of the present invention, both of the band at the position of 204 nts indicating the RNA amplified product of the target sequence and the band at the position of 226 nts indicating the DNA amplified product of the target sequence could be observed even with a very small amount of the number of *E. coli* cells as a template being 8.6 CFU/µl. From this, it was found that the process from extraction of RNA from a sample to detection can be carried out as a "one-pot" reaction using the isothermal gene amplification method of the present invention.

### Example 5

Conditions in (C) RNA amplifying of the isothermal gene amplification method of the present invention were examined.

5.0 µl of a substrate stock solution, 5 µl of a primer stock solution, and 2.5 µl of an antisense RNA genomic sample (20 ng (0.87 × 10¹¹)) prepared in Example 1 (1) were mixed in a PCR tube, incubated at 65°C for 5 minutes, and then incubated at 41°C for another 5 minutes to prepare a reaction mixture. The substrate stock solution was composed of 17.6 µl of nuclease free water (NFW), 5.6 µl of 1M MgCl₂, 19.8 µl of 1.9M Tris HCl (pH8.6), 0.6 µl of 40U/µl RNase inhibitor, 3.3 µl of 100 mM DTT, 41.3 µl of 2.0 mM dNTP, 17.1 µl of 58 mM NTP, and 4.8 µl of 250 mM ITP. The primer stock solution was composed of 32.5 µl of NFW, 21.5 µl of 2M KCl, 6.6 µl of a 50µM sense forward primer (the following forward primer 1), 6.6 µl of an antisense reverse primer (the following reverse primer 2) having a 50 µM T7 promoter in the 5'-side sequence, and 42.9 µl of dimethyl sulfoxide. Hereinafter, the combination of primers 1 and 2 is also referred to as the primer set 1. At the same time, carried out was a test system using the sense genomic sample instead of the antisense genomic sample and using the primer stock solution containing the combination of the following forward primer 3 and reverse primer 4 (primer set 2) instead of the primer set 1. In the base sequences of the primers 1 to 4 below, the underlined sequences are T7 promoter sequences.
Primer set 1:
   Forward primer 1 (SEQ ID NO: 7)
      5'-CACATTGGCA CCCGCAATC-3'
   Reverse primer 2 (SEQ ID NO: 8)
      5'-AATTCTAATACGACTCACTATAGGGAGAGAGGAACGAGAAGAGGCTTG-3'
Primer set 2:
   Forward primer 3 (SEQ ID NO: 9)
      5'-AATTCTAATACGACTCACTATAGGGAGACACATTGGCACCCGCAATC-3'
   Reverse primer 4 (SEQ ID NO: 10)
      5'-GAGGAACGAGAAGAGGCTTG-3'

Then, 2.5 µl of an enzyme stock solution was added to the reaction mixture to start the reaction, and the reaction was performed at 41°C for 10 to 30 minutes. The enzyme stock solution was composed of 4.0 µl of 10 mg/ml bovine serum albumin, 4.4 µl of 20,000 unit/ml AMV Reverse Transcriptase (Life Sciences Advanced Technologies, Inc., Petersburg, FL), 11.1 µl of 60% Sorbitol, and 35.5 µl of a T7 RNA polymerase solution (TOYOBO CO., LTD.). The amplified product was then analyzed with a 1-inch 6% denatured polyacrylamide gel (ref) and SYBR^{™} Gold Nucleic Acid Gel Stain.

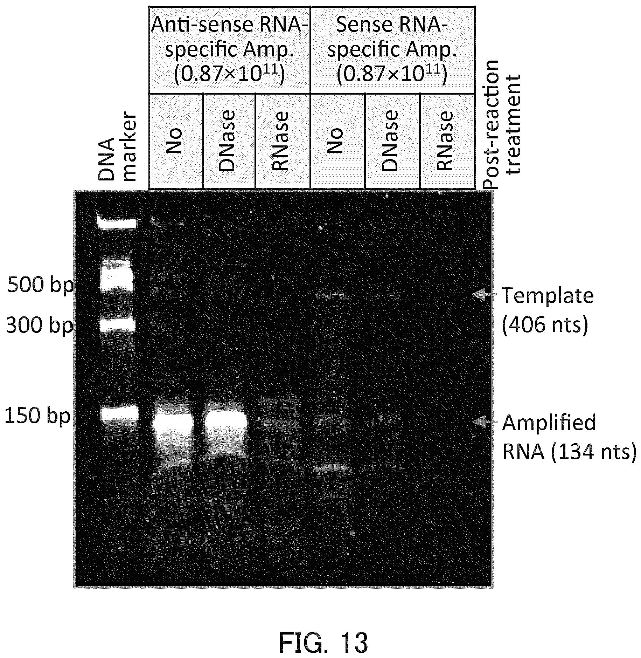

The results are shown in FIG. 13. FIG. 13 is a photograph showing a gel image of the amplified products after the reaction. In FIG. 13, the leftmost lane shows the DNA marker, the second to the fourth lanes from the left show the results of amplifying the antisense RNA samples by the primer set 1, and the fifth to the seventh lanes from the left show the results of amplifying the sense RNA samples by the primer set 2. In the lanes showing the primer set 1 and the primer set 2, whether or not the post-reaction treatment has been performed are shown, and the lanes in order from the left show the result with no DNase or RNase treatment, the result with DNase treatment, and the result with RNase treatment. As shown in FIG. 13, regardless of the target being antisense genomic RNA or sense genomic RNA, a clear band was observed at the position of 134 nts, and the amplification of the target sequence was observed. In the amplification of the antisense RNA sample, a clear band was observed at the position of 134 nts, and a significant amplification result of the target sequence was observed. On the other hand, in the amplification of the sense RNA sample, although the amplification of the target sequence was observed, the reaction result was weaker than the amplification result of the antisense RNA sample. This suggested that, in the gene amplification method of the present invention, it is preferable, for example, that the target RNA genome be the sense strand, the first primer be the antisense primer, and the second primer be the sense primer.

Next, the limit of detection (LOD) by the antisense RNA and the primer set 1 was examined in the same manner as described above except that the antisense RNA and the primer set 1 were used and the antisense RNA was serially diluted from 20 ng (0.87 × 10¹¹) to 20 fg (0.87 × 10⁵).

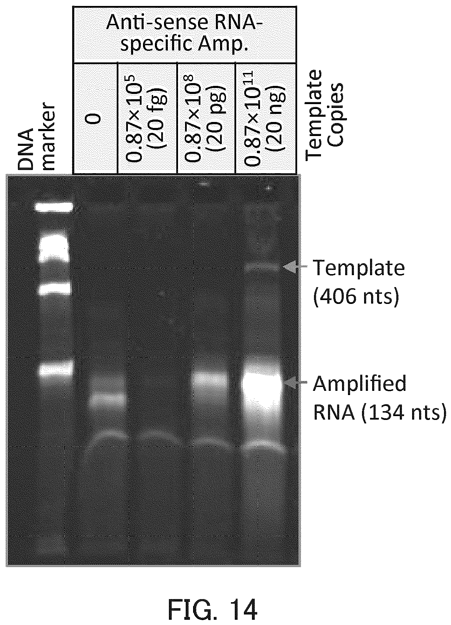

The results are shown in FIG. 14. FIG. 14 is a photograph showing a gel image of the amplified products after the reaction. In FIG. 14, the leftmost lane shows the DNA marker, the second lane from the left shows the result without antisense RNA (0 ng), the third lane from the left shows the result with 20 fg (0.87 × 10⁵) of antisense RNA, the fourth lane from the left shows the result with 20 pg (0.87 × 10⁸) of antisense RNA, and the fifth lane from the left shows the result with 20 ng (0.87 × 10¹¹) of antisense RNA. As shown in FIG. 14, in the amplification results of the antisense RNA samples by the RNA-specific amplification assay, it was found that the case where the amount of the target antisense RNA was 20 fg (0.87 × 10⁵) was the limit of detection.

Next, the amplification result was examined in the same manner as described above except that the antisense RNA and the primer set 1 were used, and the amount of the antisense RNA was set to 20 ng (0.87 × 10¹¹), and the reaction temperature was changed from 37°C to 43°C, and the reaction time was changed from 10 minutes to 30 minutes.

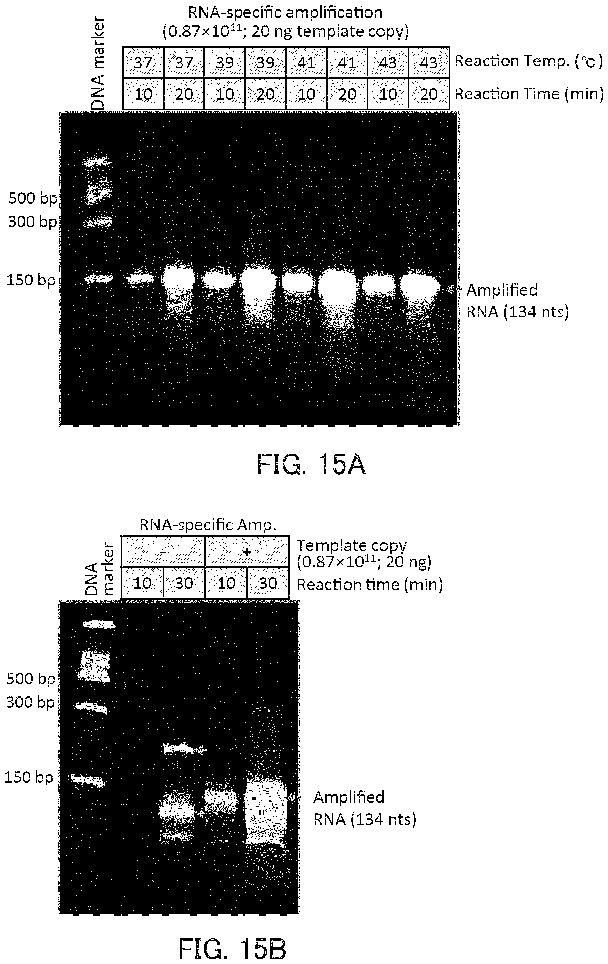

The results are shown in FIGs. 15A and 15B. FIGs.15A and 15B are photographs each showing a gel image of the amplified products after the reaction. In FIG. 15A, the leftmost lane shows the DNA marker, and the number above each lane indicates the combination of the reaction temperature (Reaction Temp. (°C)) and the reaction time (Reaction Time (min)). As shown in FIG. 15A, since the band of the target sequence at 134 nts is thickened by changing the reaction time from 10 minutes to 20 minutes at any reaction temperature, it was found that the amplification efficiency increased as reaction time increased. Further, it was found that the reaction temperature of 41°C was suitable for the amplification of the RNA genome of SARS-CoV-2.

FIG. 15B is a gel image showing the difference in the result with and without a genomic sample with a reaction time of 10 minutes or 30 minutes at a reaction temperature of 41°C, the leftmost lane shows the DNA marker, the second and third lanes from the left show the results without an RNA genomic sample (negative control) with the reaction time of 10 minutes or 30 minutes, and the third and fourth lanes from the left show the results with an RNA genomic sample with the reaction time of 10 minutes or 30 minutes. As shown in FIG. 15B, when the reaction time exceeded 30 minutes, non-specific sequences were amplified in the lanes of the negative control without an RNA genomic sample.

Next, as the antisense RNA, a genomic RNA of another coronavirus (CoV-229E) was used instead of the SARS-CoV-2 genome, and the antisense RNA was serially diluted from 20 pg (0.87 × 10⁷) to 20 fg (0.87 × 10⁵).

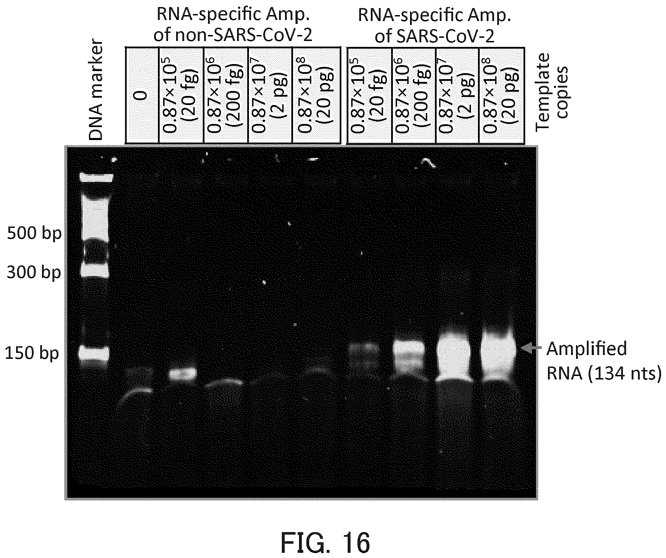

The results are shown in FIG. 16. FIG. 16 is a photograph showing a gel image of the amplified products after the reaction. In FIG. 16, the leftmost lane shows the DNA marker, the second lane from the left shows the result of the negative control, the third to the sixth lanes from the left show the results with Cov-229E RNA genome, and the seventh to the tenth lanes from the left show the results with the RNA genome of SARS-CoV-2. In the lanes showing the results of the respective RNA genomic samples, the lanes in order from the left show the result with the amount of the RNA genomic sample being 20 fg (0.87 × 10⁵), the result with the amount of the RNA genomic sample being 200 fg (0.87 × 10⁶), the result with the amount of the RNA genomic sample being 2 pg (0.87 × 10⁶), and the result with the amount of the RNA genomic sample being 2 pg (0.87 × 10⁷). As shown in FIG. 16, with the primer set 1, a specific band (134 nts) indicating a target sequence could not be observed in Cov-229E genome, whereas a specific band (134 nts) indicating a target sequence could be observed in the RNA genome of SARS-CoV-2 even with the amount of the RNA genomic sample being 20 fg (0.87 × 10⁵). From this, it was found that the primer set 1 allows specific amplification of the RNA genome of SARS-CoV-2.

Next, the cross-reactivity between the primer set 1 and the primer set 3 with SARS-CoV-2 genomic RNA and CoV-229E genomic RNA was examined. The RNA-specific amplification was performed in the same manner as described above except that the primer set 1 or the primer set 3 was used as a primer set and SARS-CoV-2 genomic RNA or CoV-229E genomic RNA was used as a genomic RNA sample.

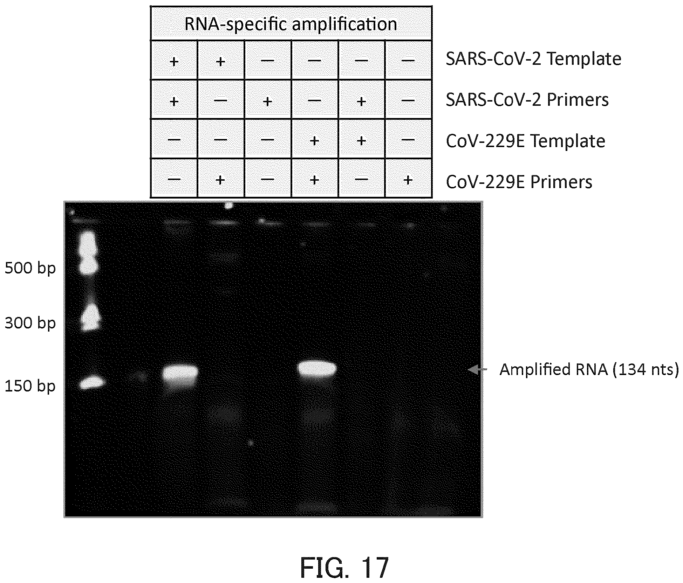

The results are shown in FIG. 17. FIG. 17 is a photograph showing a gel image of the amplified products after the reaction. In FIG. 17, the leftmost lane shows the DNA marker. As to the lanes other than the lane showing the DNA marker, "+" in the upper table means that the primer set or the genomic sample was added, and "-" means that the primer set or the genomic sample was not added. As shown in FIG. 17, the primer set 1 for amplifying the target sequence of SARS-CoV-2 amplified only an RNA genomic sample of SARS-CoV-2, and the primer set 3 for amplifying the target sequence of Cov-229E amplified only an RNA genomic sample of Cov-229E. From these results, it was found that the primer set 1 and the primer set 3 used in Examples had high specificity for SARS-CoV-2 or Cov-229E and did not exhibit cross-reactivity with each other.

Next, the amplification result was examined in the same manner as described above except that the antisense RNA and the primer set 1 were used, the amount of the antisense RNA was set to 20 ng (0.87 × 10¹¹), and saliva was added to the reaction container.

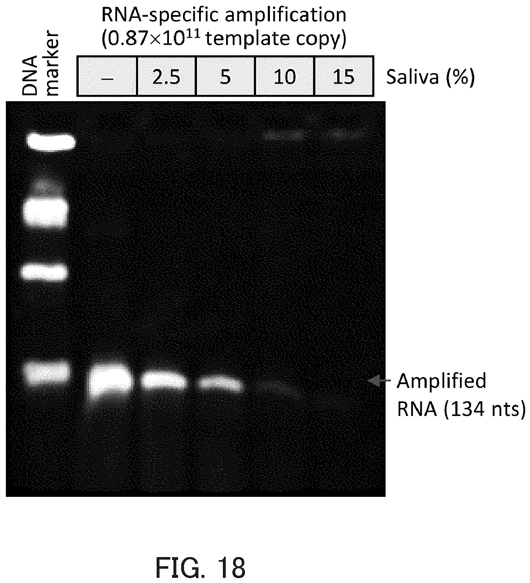

The results are shown in FIG. 18. FIG. 18 is a photograph showing a gel image of the amplified products after the reaction. In FIG. 18, the leftmost lane shows the DNA marker, the second lane from the left shows the result without the saliva sample, the third lane from the left shows the result obtained by adding the saliva sample at 2.5% relative to the total weight of the reaction solution, the fourth lane from the left shows the result obtained by adding the saliva sample at 5% relative to the total weight of the reaction solution, the fifth lane from the left shows the result obtained by adding the saliva sample at 10% relative to the total weight of the reaction solution, and the sixth lane from the left shows the result obtained by adding the saliva sample at 15% relative to the total weight of the reaction solution. As shown in FIG. 18, it was suggested that the target RNA can be amplified and detected by (C) RNA amplifying of the amplification method of the present invention even if the saliva sample was mixed at 10% relative to the total weight of the reaction solution.

### Example 6

It was examined that heat-inactivated coronavirus SARS-CoV-2 (ATCC VR-1986HK) in a saliva sample can be detected by the isothermal gene amplification method of the present invention.

First, saliva samples were collected from the subjects. Specifically, a saliva oral swab (SOS) was put into the oral cavity of the subject and placed sublingually, and allowed to wait for one minute. The SOS was then collected from the subject and placed in the barrel of a syringe with a 0.45-µm filter. The SOS in the barrel was squeezed using a plunger and 50 µl of saliva sample was transferred to Tube-1 containing 2.5 µl of Proteinase K. Tube-1 was allowed to stand at room temperature for 2 minutes with agitation, and then incubated at 95°C for 3 minutes. 50 µl of an RNA extraction reagent (0.1% Triton X-100, 2X phosphate-buffered saline pH 7.4) was then added to Tube-1 and Tube-1 was incubated for 5 minutes at room temperature with agitation, thereby preparing a saliva sample.

The isothermal gene amplification method of the present invention was performed in the same manner as in Example 1 (2) and (3), except that the RNA genomic sample of SARS-CoV-2 prepared in Example 1(1) was serially diluted to predetermined concentrations (8000 copies/µl, 4000 copies/µl, 1600 copies/µl, 800 copies/µl, 2100 copies/µl, 210 copies/µl, 21 copies/µl, and 2.1 copies/µl) and added to the 10% saliva sample to detect the RNA genomic sample of SARS-CoV-2 contained in the saliva sample. As a control, the isothermal gene amplification method of the present invention was performed in the same manner as described above except that the saliva sample containing no RNA genomic sample was used. As the comparative example, an RNA genomic sample was amplified in the same manner as in Example 1 (2) except that 28000 copies/µl of the RNA genomic sample was added to the 10% saliva sample.

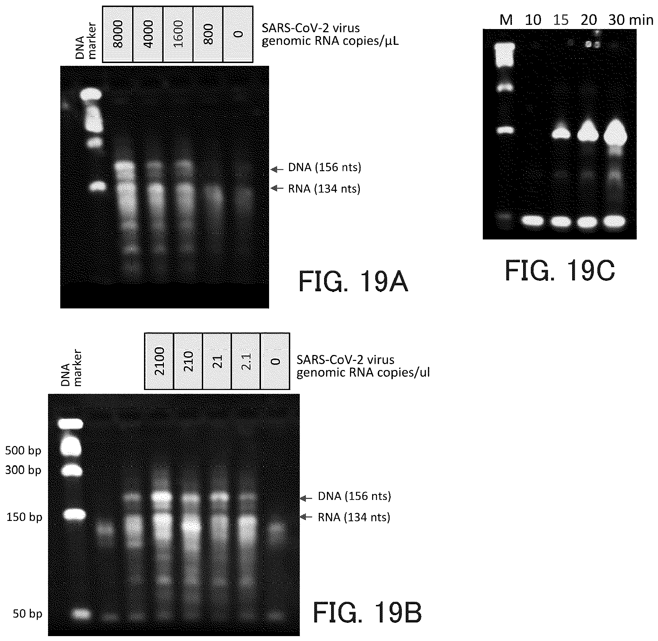

The results are shown in FIGs. 19A to 19C. FIGs. 19A to 19C are photographs each showing a gel image of the amplified products after the reaction. FIG. 19A shows the results obtained by the isothermal gene amplification method of the present invention when the concentration of the RNA genomic sample was 8000 copies/µl, 4000 copies/µl, 1600 copies/µl, or 800 copies/µl, FIG. 19B shows the results obtained by the isothermal gene amplification method of the present invention when the concentration of the RNA genomic sample was 2100 copies/µl, 210 copies/µl, 21 copies/µl, or 2.1 copies/µl, and FIG. 19C shows the results of the comparative example. In FIGs. 19A and 19B, the leftmost lane shows the DNA marker, and the number above each lane indicates the concentration of the RNA genomic sample added to the saliva sample. In FIG. 19C, the leftmost lane shows the DNA marker, and the number above each lane indicates the reaction time. As shown in FIGs. 19A and 19B, it was found that both of the band at the position of 134 nts indicating the RNA amplified product of the target sequence and the band at the position of 156 nts indicating the DNA amplified product of the target sequence can be observed at any of the concentration of the RNA genomic sample contained in the saliva sample, and SARS-CoV-2 contained in the saliva sample can be detected according to the isothermal gene amplification method of the present invention in a short time of 10 minutes. In addition, since the RNA genome of SARS-CoV-2 contained in the saliva sample can be detected even at a very low concentration of 2.1 copies/µl, it was found that the gene detection method using the isothermal gene amplification method of the present invention has a very low limit of detection (LOD) of the genomic RNA and allows a target sequence to be detected with high sensitivity. Further, as shown in FIG. 19C, in the RNA-specific amplification method of the comparative example, the target sequence could not be detected in a 10-minute reaction even when the concentration of the RNA genomic sample contained in the saliva sample was as high as 28000 copies/µl.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

### Industrial Applicability

According to the present invention, it is possible to easily and inexpensively detect a pathogen such as a virus of an infectious disease in a short time. Therefore, the present invention is extremely useful in the medical field, the public health field, and the like.

## Claims

1. An isothermal gene amplification method of a target sequence in an RNA genome, comprising:
(A) producing an amplification reaction template, comprising:
(A1) hybridizing, a first primer having a promoter sequence of an RNA polymerase on a 5' side of the first primer and a complementary sequence that is complementary to a 5'-side sequence of the target sequence in the RNA genome on a 3' side of the first primer, to the 5'-side sequence of the target sequence in the RNA genome, and extending a 3' end of the first primer by reverse transcription activity of a reverse transcriptase so as to produce a double-stranded complex of the RNA genome and an extended DNA strand;
(A2) degrading the RNA genome of the double-stranded complex formed in (A1) by RNA degrading activity of the reverse transcriptase so as to produce a single-stranded DNA having a first primer sequence; and
(A3) hybridizing a second primer having the same sequence as a 3'-side sequence of the target sequence to a sequence complementary to the target sequence of the single-stranded DNA produced in (A2), and extending a 3' end of the second primer by DNA synthetic activity of the reverse transcriptase so as to produce a single-stranded DNA having a second primer sequence, thereby producing a double-stranded DNA as the amplification reaction template;
(B) DNA amplifying for increasing the amplification reaction template, comprising:
(B1) hybridizing the second primer to a 3' side of the single-stranded DNA comprising the first primer and hybridizing the first primer to a 3' side of the single-stranded DNA having the second primer sequence in the double-stranded DNA as the amplification reaction template produced in (A3) by a recombinase in a presence of a single-stranded DNA-binding protein; and
(B2) extending the 3' end of the first primer and the 3' end of the second primer by strand displacement DNA synthetic activity of a strand displacement DNA polymerase in a presence of the single-stranded DNA-binding protein so as to produce a double-stranded DNA that is the same as the amplification reaction template produced in (A3); and
(C) RNA amplifying, comprising:
(C1) synthesizing a single-stranded RNA by an RNA synthetic activity of an RNA polymerase with a sequence that is downstream on a 3'-side relative to the promoter sequence of an RNA polymerase of the double-stranded DNA as the amplification reaction template produced in (A3) being used as a template sequence;
(C2) hybridizing the second primer to a 5' side of the single-stranded RNA synthesized in (C1), and extending the 3' end of the second primer by the reverse transcription activity of the reverse transcriptase so as to produce a double-stranded complex of the single-stranded RNA and an extended DNA;
(C3) degrading the single-stranded RNA of the double-stranded complex produced in (C2) by the RNA degrading activity of the reverse transcriptase so as to produce a single-stranded DNA; and
(C4) hybridizing the first primer to a 3' side of the single-stranded DNA produced in (C3), extending the 3' end of the first primer by the DNA synthetic activity of the reverse transcriptase, and extending a 3' end of the single-stranded DNA produced in (C3) with the promoter sequence of the RNA polymerase of the first primer being used as a template so as to produce a double-stranded DNA that is the same as the amplification reaction template produced in (A3).

2. The isothermal gene amplification method according to claim 1, wherein
the promoter sequence of the RNA polymerase included in the first primer is a T7 promoter sequence, and
the RNA polymerase is a T7RNA polymerase.

3. The isothermal gene amplification method according to claim 1-or 2, wherein
the RNA genome is a sense strand, the first primer is an antisense primer, and the second primer is a sense primer.

4. A kit for use in the isothermal gene amplification method according to any one of claims 1 to 3, comprising:
(a1) the reverse transcriptase;
(a2) the first primer;
(a3) the second primer;
(b1) the recombinase;
(b2) the single-stranded DNA-binding protein;
(b3) the strand displacement DNA polymerase; and
(c1) the RNA polymerase.

5. A gene detection method for detecting a gene of a target sequence of an RNA genome, comprising:
amplifying a gene; and
detecting an amplified gene, wherein
the amplifying is performed by the isothermal gene amplification method according to any one of claims 1 to 3.

6. The gene detection method according to claim 5, wherein
in the amplifying, one primer selected from the group consisting of the first primer and the second primer is a primer having an antigen capable of specifically binding to an immobilized antibody immobilized on a substrate incorporated at a 5' end of the one primer, and the other primer of the first primer or the second primer that is not the one primer is a primer having a first conjugate incorporated at a 5' end of the other primer,
in the detecting, the antigen of the one primer of a gene amplified product produced in the amplifying binds to the immobilized antibody, thereby immobilizing the gene amplified product to the substrate, the first conjugate of the other primer binds to a labeling substance comprising a second conjugate that specifically binds to the first conjugate via the second conjugate, thereby immobilizing the labeling substance to the substrate, and the labeling substance immobilized on the substrate is detected.

7. The gene detection method according to claim 6, wherein
the first conjugate is biotin, the second conjugate is streptavidin, and the labeling substance is a colored fine particle having the streptavidin immobilized on its surface.

8. A kit for use in the gene detection method according to claim 6 or 7, comprising:
(a1) the reverse transcriptase;
(a2) the first primer;
(a3) the second primer;
(b1) the recombinase;
(b2) the single-stranded DNA-binding protein;
(b3) the strand displacement DNA polymerase;
(c1) the RNApolymerase;
(d1) the immobilized antibody;
(d2) a substrate; and
(d3) a labeling substance.

9. The gene detection method according to claim 5, wherein
the detecting is performed by applying a voltage to a working electrode and a counter electrode and measuring a current between the working electrode and the counter electrode in a presence of a redox substance that binds to a double-stranded site of an amplified product of the target sequence amplified in the amplifying.

10. The gene detection method according to claim 9, wherein
in the detection, when a current value between the working electrode and the counter electrode is higher than a predetermined reference current value within a specific voltage, a positive determination is made that a target sequence nucleic acid is present in the genome, and when the current value between the working electrode and the counter electrode is lower than the predetermined reference current value within a specific voltage, a negative determination is made that the target sequence nucleic acid is not present in the genome.

11. The gene detection method according to claim 9 or 10, wherein
in the detecting, the current between the working electrode and the counter electrode is measured in a presence of a probe nucleic acid capable of complementary binding to the amplified product specifically, and
the probe nucleic acid is a probe nucleic acid having a magnetic fine particle with a surface coated with gold bound at one end and having the redox substance bound at the other end.

12. The gene detection method according to any one of claims 9 to 11, wherein
the redox substance is methylene blue.

13. A kit for use in the gene detection method according to any one of claims 9 to 12, comprising:
(a1) the reverse transcriptase;
(a2) the first primer;
(a3) the second primer;
(b1) the recombinase;
(b2) the single-stranded DNA-binding protein;
(b3) the strand displacement DNA polymerase;
(c1) the RNApolymerase;
(d1) the working electrode and the counter electrode; and
(d2) the redox substance or a nucleic acid probe that binds to the double-stranded site of the amplified product.

14. An RNA virus detection method being a method for detecting a sequence specific to the RNA virus in a viral genome as a target sequence, wherein
the detection of the target sequence is performed by the gene detection method according to claim 5, 6, 7, 9, 10, 11, or 12.

15. Akit for use in the RNA virus detection method according to claim 14, comprising:
the kit according to claim 8 or 13.
